# EUROPÄISCHE PATENTSCHRIFT

(11) **EP 1 960 002 B1**
(45) Veröffentlichungstag und Bekanntmachung des Hinweises auf die Patenterteilung: **02.11.2011**
(21) Anmeldenummer: 06830457.5
(22) Anmeldetag: 07.12.2006
(51) Int. Cl.: A61K 49/18

(54) **WÄSSRIGE DISPERSION VON SUPERPARAMAGNETISCHEN EINDOMÄNENTEILCHEN, DEREN HERSTELLUNG UND VERWENDUNG ZUR DIAGNOSE UND THERAPIE**
AQUEOUS DISPERSIONS OF SUPERPARAMAGNETIC SINGLE DOMAIN PARTICLES PRODUCTION AND USE THEREOF FOR DIAGNOSIS AND THERAPY
DISPERSIONS AQUEUSES DE PARTICULES SUPERPARAMAGNETIQUES MONODOMAINE, LEUR PRODUCTION ET LEUR UTILISATION DANS LE DIAGNOSTIC ET LA THERAPIE

(30) Priorität: 09.12.2005 DE 102005059751
(43) Veröffentlichungstag der Anmeldung: 27.08.2008
(73) Patentinhaber: Pilgrimm, Helga, 14193 Berlin (DE)
(72) Erfinder: PILGRIMM, Herbert, 14193 Berlin (DE)
(74) Vertreter: Ziebig, Marlene
(86) Internationale Anmeldenummer: PCT/EP2006/069453
(87) Internationale Veröffentlichungsnummer: WO 2007/065935

(56) Entgegenhaltungen:
- EP-A2- 0 284 549
- WO-A-2004/034411
- DE-A1- 19 612 001
- US-A- 4 714 607
- WAGNER S ET AL: "Monomer-coated very small superparamagnetic iron oxide particles as contrast medium for magnetic resonance imaging: Preclinical in vivo characterization" INVESTIGATIVE RADIOLOGY 2002 UNITED STATES, Bd. 37, Nr. 4, 2002, Seiten 167-177, XP009082827 ISSN: 0020-9996
- SUN Y ET AL: "An improved way to prepare superparamagnetic magnetite-silica core-shell nanoparticles for possible biological application" JOURNAL OF MAGNETISM AND MAGNETIC MATERIALS, ELSEVIER SCIENCE PUBLISHERS, AMSTERDAM, NL, Bd. 285, Nr. 1-2, Januar 2005 (2005-01), Seiten 65-70, XP004659948 ISSN: 0304-8853
- SCHNORR J ET AL: "Comparison of the iron oxide-based blood-pool contrast medium VSOP-C184 with gadopentetate dimeglumine for first-pass magnetic resonance angiography of the aorta and renal arteries in pigs" INVESTIGATIVE RADIOLOGY 2004 UNITED STATES, Bd. 39, Nr. 9, 2004, Seiten 546-553, XP009082829 ISSN: 0020-9996

## Beschreibung

Die Erfindung betrifft eine wässrige Dispersion von superparamagnetischen eisenhaltigen Teilchen, die auf ihrer Oberfläche als Stabilisatorsubstanzen α-Hydroxycarbonsäuren tragen, wobei die Dispersion N-Methyl-D-glucamin (Meglumin) und/oder 2-Amino-2-(hydroxymethyl)-1,3-propandiol (Trometamol) umfasst und der freie Eisenionengehalt kleiner 1 mg/l Eisen ist. Die erfindungsgemäße Dispersion kann in einer bevorzugten Ausführungsform zusätzlich einen Komplexbildner für Eisen enthalten. In einer weiteren bevorzugten Ausführungsform beinhaltet die Dispersion positiv geladene Metallionen und/oder polyaminogruppenhaltige Verbindungen, die an therapeutisch oder diagnostisch wirksame Substanzen gebunden sein können.

Gegenstand der Erfindung ist auch das Herstellungsverfahren dieser Dispersion, deren Verwendung als MRT-Kontrastmittel sowie deren Verwendung als Therapeutikum, auch mit der Möglichkeit der Verlaufskontrolle der Therapie durch ein Bildgebungsverfahren.

In den letzten Jahren gewinnt die ,Molekulare Bildgebung', also die in vivo-Charakterisierung und Darstellung von biologischen Prozessen auf dem zellulären und molekularen Niveau, bei der Erforschung von Krankheiten und zunehmend auch bei der klinischen Anwendung immer mehr an Bedeutung. Grundlage dafür ist die Entwicklung von molekularen Markern, die mit den zur Verfügung stehenden oder zu entwickelnden Bildgebungstechniken die gewünschten molekularen Targets ausreichend sensitiv detektieren können.

Die Magnetresonanztomographie (MRT) hat sich auf Grund ihres im Vergleich zu anderen Bildgebungstechniken hervorragenden Weichteilkontrastes bei gleichzeitig hoher anatomischer Auflösung als wichtige Säule der klinisch-radiologischen Diagnostik etabliert. Mit der Einführung superparamagnetischer Eisenoxid-Nanopartikel stehen aufgrund ihrer hohen T2- und teilweise T1-Relaxivität wirksame Marker für die ,Molekulare Bildgebung' zur Verfügung.

In den Patentanmeldungen WO-A-96/03653, WO-A-97/35200 und WO-A-2004/034411 sind sehr kleine superparamagnetische Eisenoxidpartikel, genannt VSOP (Very Small Iron Oxide Particles), beschrieben, die sich für ein molekulares Imaging und für das drug targeting gut eignen.

VSOP sind im Vergleich zu den vorher bekannten polymerbeschichteten (z. B. mit Dextran) superparamagnetischen Eisenoxidpartikel (SPIO, USPIO) deutlich kleiner. Beispielsweise weisen Citrat-beschichtete VSOP einen hydrodynamischen Durchmesser von ~7 nm auf, während die kleinsten polymerummantelten USPIO einen Durchmesser von etwa 15 - 20 nm haben.

Allerdings weisen auch diese bisher bekannten kleinen superparamagnetischen Eisenpartikel keine optimale Verträglichkeit bei parenteraler oder enteraler Anwendung im tierischen oder menschlichen Körper auf.

Dreiwertige und insbesondere zweiwertige Eisenionen sind hochtoxisch für biologisches Gewebe sowie für Säugetiere und Menschen. So wurde gefunden, dass die Toxizität von mit Citronensäure stabilisierten Mangan- Eisenferriten sehr hoch ist (Lacava et. al, Biological effects of magnetic fluids: toxicity studies, J of Magnetism a. Magnetic Materials, 201 (1999) 431-434).

Dies ist auch aus der Anwendung von Eisenkomplexen für die parenterale Eisenerzsatz-Therapie bei Eisenmangelanämie bekannt. So führt die intravenöse Injektion von einem Eisen-Sucrose-Komplex als Wirkstoff in dem fertigen zugelassenen Arzneimittel Venofer^{®} zu einer vorübergehenden Nierenschädigung, welche durch oxidativen Stress durch freie Eisenionen ausgelöst wird (Agarwal et. al, Kidney International, 2004 Vol. 65: 2279-2289). Des weiteren wirken sich freie Eisenionen toxisch auf rote Blutkörperchen aus (Gefahr der Hämolyse).

Neben der direkt zellschädigenden Wirkung freier Eisenionen gibt es bei den bekannten klinisch zugelassenen Präparaten zur Eisenersatztherapie und auch bei den klinisch zugelassen Eisenoxidpartikeln für die MR-Diagnostik das Nebenwirkungsspektrum der anaphylaktischen Reaktion, hervorgerufen durch polymere Stabilisatorsubstanzen, wie z. B. das Dextran.

Das nicht hitzestabilisierbare Endorem^{®} (AMI 227) von Laboratoire Guerbet (Frankreich) wurde zwar auf der Basis von superparamagnetischen Eisenoxid-Nanopartikeln entwickelt, ist aber mit Dextran stabilisiert und daher sehr unverträglich. Wegen der Unverträglichkeit darf es nur durch Infusion mit einer Glucoselösung und in einer geringen Konzentration à 20 µmol Fe/kg n Eisen eingesetzt werden. Es ist zugelassen für den Nachweis von Lebertumoren durch MRT.

Ein weiteres zugelassenes leberspezifisches superparamagnetisches Eisenoxidpartikel ist Resovist^{®} von der Schering AG (Deutschland). Dabei handelt es sich um relativ große, dextranummantelte superparamagnetische Eisenoxidpartikel, die nach Applikation sofort von den Makrophagen der Leber aufgenommen werden. Diese Partikel zirkulieren also nur sehr kurze Zeit im Blut. Trotz der niedrigen Dosierung von 20 µmol Fe/kg kann es zu Unverträglichkeiten kommen.

Aufgabe der Erfindung war es deshalb, eine wässrige Dispersion von sehr kleinen superparamagnetischen eisenhaltigen Teilchen zur Verfügung zu stellen, die einen hohen Kontrasteffekt liefert, aber weniger toxisch ist, so dass auch eine parenterale Anwendung ohne Nebenwirkungen möglich ist. Auch soll die Dispersion hitzesterilisierbar sein, ohne dass die freie Eisenionenkonzentration wesentlich ansteigt und ohne dass die Wirksamkeit der eisenhaltigen Teilchen verloren geht und eine Verschlechterung des Kontrastes eintritt. Daneben sollen die eisenhaltigen Teilchen auch eine längere Verweildauer im Blut aufweisen.

Die Aufgabe der Erfindung wird gemäß den Patentansprüchen gelöst. Die Unteransprüche stellen bevorzugte Ausführungsformen der zugehörigen unabhängigen Ansprüche dar. Demgemäß wird eine wässrige Dispersion von Eindomänenteilchen aus Eisenhydroxid, Eisenoxidhydrat, Eisenoxid, Eisenmischoxid oder Eisen mit einer Teilchengröße von 2 bis 10 nm, die auf ihrer Oberfläche als Stabilisatorsubstanzen aliphatische Di- und/oder Tricarbonsäuren ausgewählt aus Zitronensäure, Äpfelsäure, Weinsäure oder deren Gemischen tragen, zur Verfügung gestellt, die dadurch gekennzeichnet ist, dass sie N-Methyl-D-glucamin (Meglumin) und/oder 2-Amino-2-(hydroxymethyl)-1,3-propandiol (Trometamol) umfasst und der freie Eisenionengehalt kleiner 1 mg/l Eisen ist. Die Dispersion ist herstellbar durch Fällung der eisenhaltigen Teilchen aus wässrigen Eisensalzlösungen mit Alkalilauge oder Ammoniumhydroxid, anschließender Behandlung mit den genannten Di- und/oder Tricarbonsäuren oder deren Gemischen und Reinigung der so stabilisierten Teilchen durch Dialyse mit destilliertem Wasser bis das Dialysat eine elektrische Leitfähigkeit kleiner 10 µS/cm besitzt. Die so erhaltene Dispersion wird als vorgereinigte Dispersion bezeichnet. Danach erfolgt die erfindungsgemäße Behandlung der vorgereinigten Dispersion mit wässrigen Lösungen von Salzen aliphatischer Di- und/oder Tricarbonsäuren, ausgewählt aus Zitronensäure, Äpfelsäure, Weinsäure oder deren Gemischen und Dialyse mit destilliertem Wasser bis das Dialysat eine elektrische Leitfähigkeit kleiner 10 µS/cm und einen freien Eisenionengehalt kleiner 1 mg/l besitzt. Anschließend wird die Dispersion mit einer wässrigen Lösung der genannten freien Di- und/oder Tricarbonsäuren oder deren Gemischen behandelt und mit destilliertem Wasser dialysiert bis das Dialysat eine elektrische Leitfähigkeit kleiner 10 µS/cm und einen freien Eisenionengehalt kleiner 1 mg/l besitzt und N-Methyl-D-glucamin (Meglumin) und/oder 2-Amino-2-(hydroxymethyl)-1,3-propandiol (Trometamol) zugesetzt.

Überraschend wurde gefunden, dass die Toxizität der wässrigen Dispersion von super-paramagnetischen Eindomänenteilchen durch eine Behandlung der vorgereinigten und stabilisierten Teilchen (wie sie beispielsweise in WO 97/35200 beschrieben sind) mit Lösungen von Tri-, Di- und Monosalzen aliphatischer Di- und oder Tricarbonsäuren und anschließender Dialyse mit destilliertem Wasser , sowie nachfolgender Behandlung mit Lösungen freier Di- und/oder Tricarbonsäuren und anschließender Dialyse mit destilliertem Wasser stark verringert werden kann. Die Konzentration an freien Eisenionen verringert sich durch diese Maßnahmen im Ultrafiltrat um Größenordnungen, so dass diese sehr kleinen erfindungsgemäßen Teilchen für die parenterale Anwendung am Menschen vorteilhaft einsetzbar sind. Durch den Zusatz von N-Methyl-D-glucamin (Meglumin) und/oder 2-Amino-2-(hydroxymethyl)-1,3-propandiol (Trometamol) wird eine zusätzliche Verringerung der Toxizität erreicht.

In einer bevorzugten Ausführungsform der Erfindung wird als aliphatische Tricarbonsäure Citronensäure eingesetzt. Als Citrate finden Tri-Natrium-Citrat und Di-Natrium-Hydrogencitrat bevorzugte Anwendung.

Die z. B. mit Citronensäure stabilisierten superparamagnetischen Eindomänenteilchen bilden in wässriger Dispersion eine stabile magnetische Flüssigkeit, die durch Dialyse gegen destilliertes Wasser von den wasserlöslichen Reaktionsprodukten, die bei der Herstellung der superparamagnetischen Eindomänenteilchen entstanden sind, vorgereinigt werden. Diese Vorgehensweise ist in WO 97/35200 beschrieben und die so gewonnenen Teilchen werden als stabilisierte und vorgereinigte Teilchen in der vorliegenden Beschreibung bezeichnet.

Erfindungsgemäß bevorzugt werden nun die vorgereinigten und stabilisierten superparamagnetischen Eindomänenteilchen zur Verringerung des freien Eisenionengehalts mit wässrigen Lösungen von Tri-, Di-, oder Mono-Salzen der Citronensäure behandelt und anschließend mit destilliertem Wasser dialysiert, danach mit einer wässrigen Lösung von freier Citronensäure behandelt und anschließend erneut mit destilliertem Wasser so lange dialysiert, bis der freie Eisenionengehalt kleiner als 0,005% der Gesamtmenge an Eisen ist.

Weiterhin wurde überraschend gefunden, dass die Verweildauer der erfindungsgemäßen superparamagnetischen eisenhaltigen Teilchen im Blut durch diese Behandlung mit Lösungen von Salzen der Di- oder Tricarbonsäuren und der freien Säuren und jeweils anschließender Dialyse mit destilliertem Wasser verlängert wird.

Erfindungsgemäß werden an die so stabilisierten eisenhaltigen Teilchen monoaminogruppenhaltige Verbindungen gebunden, ausgewählt aus D(-)-N-Methylglucamin (Meglumin) oder 2-Amino-2-(hydroxymethyl)-1,3-propandiol (Trometamol) oder deren Gemisch. Hierbei hat sich überraschenderweise gezeigt, dass der vollständige oder teilweise Ersatz der Kationen, wie Ammonium-, Natrium- oder Hydroniumionen der freien Carboxylgruppen der z. B. mit Citronensäure stabilisierten Teilchen, durch N- Methyl-D-glucamin, und/oder 2-Amino-2-(hydroxymethyl)-1,3-propandiol zu einer Verringerung der Toxizität der erfindungsgemäßen eisenhaltigen Teilchen führt.

In einer weiteren erfindungsgemäßen Ausführungsform können der Dispersion physiologisch verträgliche polyaminogruppenhaltige Verbindungen zugesetzt werden, die aus der Gruppe ausgewählt sind, die Polyethylenimine (PEI), Polyvinylamine (PVAm), PEI- und PVAm- Co-Polymere, Polylysin, Spermin, Spermidin, Protamin, Protaminsulfat, Oligopeptide, Polypeptide, Denaturierungsprodukte von Proteinen und Proteiden, wie Gelatine, Kasein-Hydrolysate, Gluteline; stickstoffhaltigen Polysaccharide, wie Mucopolysaccharide, Glykoproteide, Chitine und Gemische davon, umfasst, vorzugsweise Polyethylenimine (PEI) oder Poly-vinylamine (PVAm).

Durch den teilweisen Ersatz der Kationen, wie z. B. Ammonium-, Natrium- oder Hydroniumionen der freien Carboxylgruppen der z. B. mit Citronensäure stabilisierten eisenhaltigen Teilchen, durch polyaminogruppenhaltige Verbindungen, können diagnostisch wirksame Substanzen, zell- und gewebespezifische Bindungssubstanzen, pharmakologisch wirksame Substanzen, pharmakologisch wirksame Zellen oder zellfusionsvermittelnde Substanzen an die polyaminogruppenhaltigen Verbindungen nach bekannten Kopplungsverfahren chemisch gebunden werden. Dabei können zunächst die biologisch wirksamen Substanzen an die Polyamine gebunden und aufgereinigt werden und diese Reaktionsprodukte dann an die erfindungsgemäßen eisenhaltigen Teilchen gekoppelt werden.

Als diagnostisch wirksame Substanzen können an die Polyamine z. B. Fluoreszenzfarbstoffe für den Wellenlängenbereich von 200 bis 1200 Nanometer für die Kombination der MRT mit optischen Diagnoseverfahren gebunden werden. Als Fluoreszenzfarbstoffe kommen z. B. Fluoreszein, Rhodamin green, Texas red. sowie Gemische davon in Frage.

An die polyaminogruppenhaltigen Verbindungen können auch diagnostisch wirksame Substanzen, wie perfluorhaltige Moleküle für die Ultraschalldiagnostik gebunden werden. Als perfluorhaltige Moleküle kommen z. B. Perfluoralkylphosphat, Perfluoralkoxypolyethylenglykolphosphat , Hexanfluorphospat sowie Gemische davon in Frage.

Als diagnostisch wirksame Substanzen können an die Polyamine z. B. kurzlebige Radiopharmaka für die Kombination der MRT mit der Positronen-Emissionen-Tomographie (PET) gebunden werden. Als Radiopharmaka kommen organische Substanzen , die ¹¹C, ¹³N, ¹⁵O, ¹⁸F, ⁶⁸Ga, ⁷⁵Br, ¹²³J, enthalten, wie z. B. [¹¹C] Thymidin, [¹⁸F]Fluor-L-DOPA, [⁶⁸Ga]-anti- CD66, zur Anwendung..

An die polyaminogruppenhaltigen Verbindungen können als zell- oder gewebespezifische Bindungssubstanzen z. B. Antigene, Antikörper, Ribonucleinsäuren, Desoxyribo-nucleinsäuren, Ribonucleinsäuresequenzen, Desoxyribonucleinsäuresequenzen, Haptene, Avidin, Streptavidin, Protein A, Protein G, Anexin, Endotoxinbindende Proteine, Lectine, Selectine, Integrine, Oberflächenproteine von Organellen, Viren, Mikroben, Algen, Pilze sowie Gemische davon gebunden werden.

An die polyaminogruppenhaltigen Verbindungen können als pharmakologisch wirksame Substanzen z. B. Antitumorproteine, Enzyme, Antitumorenzyme, Antibiotika, Pflanzenalkaloide, Alkylierungsreagenzien, Antimetaboliten, Hormone und Hormonantagonisten, Interleukine, Interferone, Wachstumsfaktoren, Tumornekrosefaktoren, Endotoxine, Lymphotoxine, Integrine, Urokinase, Streptokinase, Plasminogen-Streptokinase-Aktivator-Komplex, Gewebe-Plasminogen-Aktivatoren, Desmodus-Plasminogen-Aktivatoren, Makrophagen-Aktivierungs-Körper, Antisera, Blut- und Zellbestandteile und deren Abbauprodukte und Derivate, Zellwandbestandteile von Organellen, Viren, Mikroben, Algen, Pilze und deren Abbauprodukte und Derivate, Proteaseninhibitoren, Alkylphosphocholine; radioaktive Isotope enthaltende Substanzen, Tenside, kardiovaskuläre Pharmazeutika, Chemotherapeutika, gastrointestinale Pharmazeutika, Neuropharma-zeutika sowie Gemische davon gebunden werden.

An die polyaminogruppenhaltigen Verbindungen können als pharmakologisch wirksame Zellen z. B. Organellen, Viren, Mikroben, Algen, Pilze, insbesondere Erythrozyten, Thrombozyten, Granulozyten, Monozyten, Lymphozyten, Langerhans'sche Inseln gebunden werden.

Die Bindung dieser Substanzen an die polyaminogruppenhaltigen Verbindungen ist dem Fachmann bekannt. So kann die kovalente Bindung der polyaminogruppenhaltigen Verbindungen oder deren Reaktionsprodukte mit den genannten Substanzen mit den z. B. durch Citronensäure stabilisierten erfindungsgemäßen Eindomänenteilchen beispielsweise mit Substanzen aus der Gruppe der Carbodiimide, wie z. B. 1-Ethyl-3-(3-Dimethylaminopropyl)carbodiimide (EDC), 1-Cyclohexyl-3-(2-morpholinoethyl)carbodiimide (CMC) oder Dicyclohexyl carbodiimide (DCC) geknüpft werden. So lassen sich stabile Verbindungen zwischen den Carboxylgruppen der Stabilisatormoleküle auf der Oberfläche der erfindungsgemäßen Eindomänenteilchen und den Aminogruppen der genannten Substanzen erzeugen.

Die nicht-kovalente Kopplung kann durch elektrostatische Wechselwirkungen erfolgen. Beispielsweise binden Polyamine elektrostatisch an Zitrat-beschichtete eisenhaltige Teilchen.

In einer weiteren erfindungsgemäßen Ausführungsform der Erfindung enthält die erfindungsgemäße Dispersion positiv geladene Metallionen der chemischen Elemente wie z. B. Kupfer, Silber, Gold, Eisen, Gallium Thallium, Bismut, Palladium, Rhenium, Ruthenium, Platin, Technetium, Indium, Iridium, Radium, Selen, Ytrium, Zirkon und seltenen Erden sowie Gemische davon, wobei die Metallionen auch die radioaktiven Isotope derselben chemischen Elemente sein können wie z. B. ⁵²Fe, ⁶⁷Ga, ^{99m}Tc, ¹¹³In, ¹⁸⁸Rh, ¹⁹²Ir, ¹⁹⁸Au, ²⁰¹Tl, ²²³Ra sowie Gemische davon. Auch an diese Teilchen können zusätzlich noch die genannten mono- und/oder polyaminogruppenhaltigen Verbindungen sowie diagnostisch wirksamen Substanzen, zell- und gewebe-spezifische Bindungssubstanzen, pharmakologisch wirksame Substanzen, pharmakologisch wirksame Zellen oder zellfusionsvermittelnden Substanzen gebunden sein.

In einer weiteren erfindungsgemäßen Ausführungsform der Erfindung wird die Toxizität der erfindungsgemäßen wässrigen Dispersion der eisenhaltigen Teilchen durch Zugabe eines physiologisch verträglichen Komplexbildners für Eisen zur galenischen Formulierung noch weiter verringert und es kommt überraschenderweise zu keiner Auflösung der Teilchen. Bevozugte Komplexbildner sind z. B. Glycerolphosphorsäure, Ethylendiamintetraessigsäure (EDTA), N-Hydroxyethyl-ethylendiamintriessigsäure (HEDTA), Diethylentriaminpentaessigsäure (DTPA), α-Mercaptopropionylglycin (Tiopronin), 2,3-Mercapto-1-propansulfonsäure, 30-Amino-3,14,25-trihydroxy-3,9,14,20,25-pentaazatriacontan-2,10,13,21, 24-penton-methansulfonsäure (Deferoxaminmesilat). Aufgrund der geringen Toxizität sind diese Teilchen/Dispersionen damit auch für Mehrfachanwendungen am Menschen geeignet, so z. B. zur parenteralen Eisenersatztherapie. Die Anreicherung dieser Teilchen in Organen des blutbildenden Systems (Knochenmark, Milz) führt zu einer Depotwirkung und stellt somit für Patienten mit Eisenmangelerkrankungen eine vorteilhafte Therapie dar. Die Konzentration des physiologisch verträglichen Komplexbildners für Eisen in der Dispersion liegt im Bereich von 1 bis 20 Gew.% bezogen auf den Gehalt an Eisen.

Als Kationen der säuregruppenhaltigen Komplexbildner für Eisen können Natrium, Kalium, Calcium, Magnesium, D(-)-N- Methylglucamin (Meglumin) oder 2-Amino-2-(hydroxymethyl)-1,3-propandiol (Trometamol) sowie Gemische davon eingesetzt werden. Als Komplexbildner.wird vorzugsweise Glycerolphosphorsäure oder ein Salz davon eingesetzt, besonders bevorzugt Natrium-Glycerolphosphat.

Sollen die erfindungsgemäßen eisenhaltigen Teilchen mit gebundenen positiv geladenen Metallionen auch einen Komplexbildner für Eisen, z. B. ein Glycerolphosphat tragen, so ist es wichtig, dass zunächst die positiv geladenen Metallionen gebunden werden, d. h. der Dispersion zugesetzt werden und erst bei der Herstellung der galenischen Formulierung der Komplexbildner zugesetzt wird.

Durch die Bindung von der Dispersion zugegebenen radioaktiven Metallionen, wie z. B. Technetium 99m oder Gallium 67, auf der Oberfläche der erfindungsgemäßen superparamagnetischen Eindomänenteilchen entsteht ein Kontrastmittel, das zu einer neuartigen Kombination von MRT Bildgebung mit einer Nuklearmedizinischen Bildgebung zusammengeführt werden kann. Dieses neue Bildgebungsverfahren kombiniert das große Auflösungs-vermögen der MR-Tomographie mit der hohen Empfindlichkeit der nuklearmedizinischen Bildgebungsverfahren, wie SZINTIGRAPHIE oder SPECT (Single-Photon Emission Computed Tomography).

Durch die Bindung von Farbstoffen oder kurzlebigen radioaktiven Markern können MR-Kontrastmittel für die parenterale Anwendung hergestellt werden, die eine Kombination von MRT und optischer Bildgebung oder eine Kombination von MRT und nuklearmedizinischer Bildgebung, wie PET, ermöglichen.

Es besteht auch die Möglichkeit, die zwei getrennt aufgenommenen Bilddatensätze der MRT und der optischen oder nuklearmedizinischen Bildgebung zu einem Bild zusammenzufügen und für die verbesserte Diagnose von Erkrankungen einzusetzen.

Die sehr kleinen superparamagnetischen Eindomänenteilchen der Erfindung können aus folgenden Substanzen bestehen: Eisenhydroxid, Eisenoxidhydrat, Fe₂O₃, Fe₃O₄, aus den Eisenmischoxiden der allgemeinen Formel m MO.n Fe₂O₃, worin M die zweiwertigen Metallionen Fe, Co, Ni, Mn, Be, Mg, Ca, Ba, Sr, Cu, Zn, Pt oder Gemische davon bedeutet, oder aus den Mischoxiden der allgemeinen Formel m Fe₂O₃.n Me₂O₃, worin Me die dreiwertigen Metallionen Al, Cr, Bi, seltene Erdmetalle oder Gemische davon bedeutet oder Eisen, wobei m und n in den jeweiligen Formeln ganze Zahlen von 1 bis 6 sind. So lässt sich durch die Zusammensetzung und durch die Struktur der Eindomänenteilchen deren magnetische Suszeptibilität in weiten Grenzen variieren und ein Verhältnis der Relaxivitäten R2/R1 kleiner 5 einstellen.

Gegenstand der Erfindung ist auch das Verfahren zur Herstellung einer wässrigen Dispersion von superparamagnetischen Eindomänenteilchen aus Eisenhydroxid, Eisenoxidhydrat, Eisenoxid, Eisenmischoxid oder Eisen mit einer Teilchengröße von 2 bis 10 nm, die auf ihrer Oberfläche als Stabilisatorsubstanzen aliphatische Di- und/oder Tricarbonsäuren ausgewählt aus Zitronensäure, Äpfelsäure, Weinsäure oder deren Gemischen tragen, durch Fällung der superparamagnetischen eisenhaltigen Teilchen aus wässrigen Eisensalzlösungen mit Alkalilauge oder Ammoniumhydroxid, anschließender Behandlung mit aliphatischen Di- und/oder Tricarbonsäuren ausgewählt aus Zitronensäure, Äpfelsäure und Weinsäure oder deren Gemischen und Reinigung der so stabilisierten Teilchen durch Dialyse mit destilliertem Wasser bis das Dialysat eine elektrische Leitfähigkeit von kleiner 10 µS/cm besitzt, das dadurch gekennzeichnet ist, dass die Dispersion nachfolgend mit einer wässrigen Salzlösung von aliphatischen Di- und/oder Tricarbonsäuren, ausgewählt aus Zitronensäure, Äpfelsäure und Weinsäure oder deren Gemischen behandelt und mit destilliertem Wasser dialysiert wird bis die Leitfähigkeit des Dialysats kleiner 10 µS/cm ist und der freie Eisenionengehalt kleiner 1 mg/l ist, dann das Dialysat mit einer wässrigen Lösung der freien Di- und/oder Tricarbonsäuren, ausgewählt aus Zitronensäure, Äpfelsäure, Weinsäure oder deren Gemischen behandelt und mit destilliertem Wasser wiederum bis zu einer Leitfähigkeit des Dialysats von kleiner 10 µS/cm und bis zu einem freien Eisenionengehalt kleiner 1 mg/l dialysiert wird und N-Methyl-D-glucamin (Meglumin) und/oder 2-Amino-2-(hydroxymethyl)-1,3-propandiol (Trometamol) zugesetzt wird.

Die sehr kleinen superparamagnetischen Eindomänenteilchen werden zunächst in bekannter Weise durch Fällung aus wässrigen Eisensalzlösungen mit Alkalilauge oder Ammoniakwasser und anschließender Behandlung mit 20 bis 50 Gew.-% stabilisierender Säure, ausgewählt aus Äpfelsäure, Weinsäure, Zitronensäure oder deren Gemischen , die eine Aggregation und Sedimentation im Schwerefeld verhindern, hergestellt und nachfolgend durch Dialyse mit destilliertem Wasser bis zu einer elektrischen Leitfähigkeit des Dialysates kleiner als 10 µS/cm vorgereinigt. Durch die erfindungsgemäße Behandlung der so vorgereinigten super-paramagnetischen Eisenoxidpartikel mit vorzugsweise Lösungen von Tri-, Di-, und Mono-Salzen der Citronensäure und Dialyse mit destilliertem Wasser bis zu einem freien Eisenionengehalt kleiner 1 mg/l und anschließender Behandlung mit einer wässrigen Lösung der freien Citronensäure und Dialyse mit destilliertem Wasser bis zu einem freien Eisenionengehalt kleiner 1 mg/l wird der Anteil an freien Eisenionen auf kleiner als 0,005% der Gesamtmenge an Eisen verringert. Der Zusatz von N-Methyl-D-glucamin (Meglumin) und/oder 2-Amino-2-(hydroxymethyl)-1,3-propandiol (Trometamol) bewirkt eine weitere Verringerung der Toxizität.

So lassen sich stabilisierte superparamagnetische Eindomänenteilchen mit einem Bereich des mittleren Teilchendurchmessers d50 von 2 bis 4 nm, vorzugsweise unter 3,5 nm herstellen, die also einen mittleren hydrodynamischen Teilchendurchmessers von 5 bis 8 nm aufweisen. Das Verhältnis der Relaxivitäten R2/R1 kann so auf Werte zwischen 1 bis 3 verringert werden, vorzugsweise auf 1 bis 2. Unter "mittlerer Teilchendurchmesser d₅₀" wird verstanden, dass wenigstens 50 % der Teilchen in dem angegebenen Durchmesserbereich liegen. Die Teilchengröße wird mit Zetasizer der Fa. Malvern und mit dem Elektronenmikroskop bestimmt. Der mittlere Teilchendurchmesser bezieht sich auf die Teilchen mit (Zetasizer) und ohne Hydrathülle (Elektronenmikroskop).

Überraschend wurde gefunden, dass sehr kleine superparamagnetische Eindomänenteilchen mit einem Teilchendurchmesser kleiner 3,5 Nanometer nierengängig sind, also auch für eine MR-Diagnostik der harnableitenden Wege und vor allen Dingen für die molekulare Bildgebung geeignet sind.

Damit verlängert sich die Blut-Halbwertszeit der erfindungsgemäßen sehr kleinen superparamagnetischen Eindomänenteilchen gegenüber den bisherigen Teilchen wesentlich und die möglichen Einsatzbereiche, z. B. für die T1-gewichtete MRT-Tomographie in der Angiographie, Lymphographie, Thromben- und TumorDiagnostik, erweitern sich.

In einer bevorzugten Ausführungsform des erfindungsgemäßen Herstellungsverfahrens werden der erhaltenen wässrigen Dispersion polyaminogruppenhaltige Verbindungen zugesetzt, um Möglichkeiten für die Anbindung biologisch wirksamer Substanzen zu schaffen.

Die Bindung der polyaminogruppenhaltigen Verbindungen oder der gereinigten Reaktionsprodukte von polyaminogruppenhaltigen Verbindungen mit diagnostisch wirksamen Substanzen, zell- und gewebespezifischen Bindungssubstanzen, pharmakologisch wirksamen Substanzen, pharmakologisch wirksamen Zellen oder zellfusioniermittelnden Substanzen auf der Oberfläche der mit Säuren gegen Aggregation stabilisierten superparamagnetischen Eindomänenteilchen kann durch elektrostatische Wechselwirkungen oder über eine kovalente chemische Bindung erfolgen wie oben beschrieben.

Erfindungsgemäß kann der Dispersion auch ein Komplexbildner für Eisen zugesetzt werden, vorzugsweise eine Glycerolphosphorsäure oder ein Salz davon.

Gegenstand der Erfindung ist auch eine pharmazeutische Zusammensetzung umfassend die vorstehend definierte, erfindungsgemäße Dispersion von stabilisierten und gereinigten eisenhaltigen Teilchen, die gegebenenfalls einen Komplexbildner für Eisen und/oder gegebenenfalls positiv geladene Metallionen und/oder gegebenenfalls polyaminogruppenhaltige Verbindungen enthält. Die pharmazeutische Zusammensetzung kann pharmazeutisch annehmbare Hilfsstoffe wie z. B. Zucker, vorzugsweise Mannitol, Sorbitol, Glucose oder Xylitol, enthalten. Die Zucker sind in einer solchen Menge enthalten, dass physiologische Bedingungen, wie eine Osmolalität im Bereich von 200 bis 2000 mOs/kg, vorzugsweise von ca. 300 mOs/kg, gewährleistet sind. So enthält die pharmazeutische Zusammensetzung z. B. ca. 6% Mannitol.

Gegenstand der Erfindung ist auch die Verwendung der erfindungsgemäßen wässrigen Dispersion gemäß Anspruch 14.

Die Hauptanwendungsgebiete der erfindungsgemäßen Dispersion, die sehr kleine superparamagnetische Eindomänenteilchen enthält, liegen auf den Gebieten der MRT-Kontrastmittel für die Angiographie, Lymphographie, Thromben- und Tumordiagnostik, der Tumorschädigung, der Thrombenauflösung, der Immunsteigerung, der Zellfusionsvermittlung oder des Gentransfers, wobei auch hier die Wirksamkeit der Tumorschädigung, der Thrombenauflösung, der Zellfusion und des Gentransfers mit der MRT-Diagnostik untersucht werden kann.

Die erfindungsgemäße Dispersion, die sehr kleine stabilisierte superparamagnetische Eindomänenteilchen enthält, die mit polyaminogruppenhaltigen Verbindungen wie z. B. Pentaetylenhexamin beschichtet sind, kann für die Tumordiagnostik eingesetzt werden, da bei ihrer Injektion in den Blutkreislauf eine Anreicherung in einigen Tumorarten zu beobachten ist. Bei Kopplung von pharmakologisch wirksamen Substanzen an die sehr kleinen stabilisierten superparamagnetischen Eindomänenteilchen kann deren Konzentration am Wirkungsort erhöht werden, insbesondere bei den mit Zytostatika, wie z. B. Doxorubicin oder Paclitaxel, gebunden an ein Polyamin, wie z. B. Pentaetylenhexamin, stabilisierten sehr kleinen superparamagnetischen Eindomänenteilchen oder beim Einsatz tumorspezifischer Antikörper. Dieser Umstand hat für die Krebstherapie Bedeutung, da die zur Chemotherapie von Tumoren eingesetzten Substanzen sehr starke Nebenwirkungen auf den gesamten Organismus ausüben und bei einer Anreicherung am Wirkungsort der übrige Körper weniger stark mit Zytostatika belastet wird.

In Tierversuchen konnten für die erfindungsgemäße Dispersion gute Effekte als parenterales Positivkontrastmittel bei T1-gewichteter MR-Tomographie, wie z. B. für den Blutkreislauf, für die Thromben- und Tumordiagnostik, für die Abbildung des Magen-Darm-Traktes, sowie als antikörperspezifische Kontrastmittel, gefunden werden. Hier wirkt sich die große Bluthalbwertszeit positiv aus, da das retikuloendotheliale System die Teilchen nur langsam aufnimmt und die Teilchen, besonders wenn sie mit Antikörpern gekoppelt sind, über einen längeren Zeitraum im Blutkreislauf beweglich sind und sich so verstärkt am Bindungsort aufkonzentrieren lassen.

Bei T2-gewichteter MR-Tomographie liefert die erfindungsgemäße Dispersion noch einen guten Negativkontrast für Leber, Milz, Knochenmark und Lymphknoten.

Die Mengen der erfindungsgemäßen sehr kleinen superparamagnetischen Eindomänenteilchen liegen bei der Anwendung als parenterales Kontrastmittel für die MRT bei ca. 0,1 bis 100 µmol Fe/kg Körpergewicht und bei der Anwendung als orales Kontrastmittel für die MRI bei ca.1 bis 50 µmol Fe/kg Körpergewicht.

Die Mengen der erfindungsgemäßen sehr kleinen superparamagnetischen Eindomänenteilchen mit gebundenen radioaktiven Metallionen liegen bei der

Anwendung als parenterales Kontrastmittel für die MRT in Kombination mit SZINTIGRAPHIE, SPECT oder PET bei ca. 0,1 bis 60µmol Fe/kg Körpergewicht). Die Dosis von gebundenen radio-aktiven Metallionen, wie z. B. Technetium liegt bei der Myokardperfusion zwischen 150 und 300 MBq pro Patient und bei Gallium-67-Zitrat für die Szintigraphie bei entzündlichen Erkrankungen zwischen 100 und 220 MBq.

Die erfindungsgemäßen Teilchen und die erfindungsgemäße wässrige Dispersion sind hervorragend geeignet zur Gefäßdiagnostik als positives und negatives MR-Kontrastmittel für die magnetresonanztomographische Beurteilung des Lumens, der Wand und der morphologischen Charakterisierung von Einengungen oder Verschlüssen von Gefäßen (Arterien, Venen) des Körperstammes, der Extremitäten, der Kopf-Halsregion einschließlich intrakranieller Gefäße, von herznahen Gefäßen und Herzkranzgefäßen, zur Beurteilung der Mikrozirkulation einschließlich der Angiogenese im Rahmen von Entzündungsprozessen, Infekten oder Tumoren, zur Diagnostik entzündlich veränderter Arterienwände einschließlich verschiedener Stadien der Arteriosklerose, zur morpholo-gischen Beurteilung von Thromben oder Emboli.

Auch zur Diagnostik von Primärtumoren und deren Metastasen und zur Diagnostik des lymphatischen Systems, einschließlich der Auffindung des Sentinellymphknotens, kann die erfindungsgemäße Dispersion vorteilhaft eingesetzt werden wie in den Ausführungsbeispielen nachfolgend dargestellt ist.

Es hat sich gezeigt, dass die erfindungsgemäße Dispersion auch zur parenteralen Eisenersatztherapie eingesetzt werden kann. Dazu werden dem Patienten z. B. 20 µmol Eisen pro Woche und kg Körpergewicht i.v. verabreicht. Die Teilchen reichern sich in der Leber und den Organen des blutbildenden Systems (Knochenmark, Milz) an und werden nur langsam (sustained release) über Tage oder Wochen, je nach Teilchengröße, ins Blut abgegeben, so dass eine Depotwirkung erreicht wird.

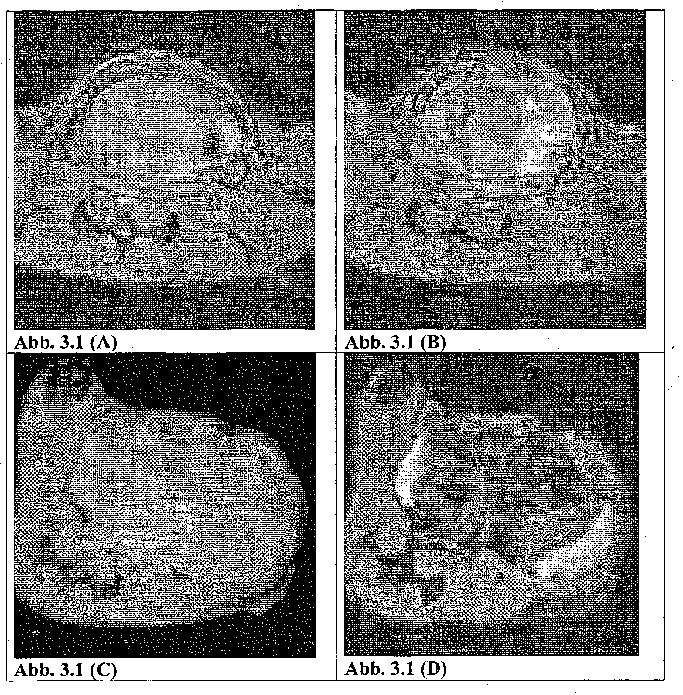

Die sehr gute Verträglichkeit und die lange Zirkulationsdauer der in der Dispersion enthaltenen gereinigten und formulierten erfindungsgemäßen Eisenoxid-Partikel ermöglicht eine Anwendung für die Tumortherapie nach intravenöser, intraarterieller und intratumoraler Injektion in Kombi-nation mit Magnetfeldern (Magnetfeldhyperthermie), Embolisaten und Chemotherapeutika. Dadurch kann eine erhöhte Anreicherung im Zielgewebe durch Bindung sogenannter targetspezifischer Liganden auf den Eisenoxid-Partikeln erreicht werden. In Abb. 3.1 ist die Verstärkung der intratumoralen Anreicherung der Partikel durch die Bindung von Polyamin auf der Oberfläche und damit die Zielrichtung an angiogenetisches Endothel dargestellt.

Die erhaltenen superparamagnetischen eisenhaltigen Teilchen aus Eisenhydroxid, Eisenoxidhydrat, Eisenoxid, Eisenmischoxid oder Eisen mit einer Teilchengröße von 2 bis 10 nm, die auf ihrer Oberfläche als Stabilisatorsubstanzen aliphatische Di- und/oder Tricarbonsäuren ausgewählt aus Zitronensäure, Äpfelsäure, Weinsäure oder deren Gemischen tragen, sind dadurch gekennzeichnet, dass sie einen freien Eisenionengehalt kleiner 0,005 % der Gesamtmenge an Eisen aufweisen und herstellbar sind durch Fällung der eisenhaltigen Teilchen aus wässrigen Eisensalzlösungen mit Alkalilauge oder Ammoniumhydroxid, anschließender Behandlung mit den Di- und/oder Tricarbonsäuren oder deren Gemischen, Reinigung der so stabilisierten Teilchen durch Dialyse mit destilliertem Wasser bis das Dialysat eine elektrische Leitfähigkeit kleiner 10 µS/cm besitzt, nachfolgender Behandlung des Dialysats mit wässrigen Salzlösungen von aliphatischen Di- und/oder Tricarbonsäuren, ausgewählt aus Zitronensäure, Äpfelsäure und Weinsäure, Dialyse mit destilliertem Wasser bis das Dialysat eine elektrische Leitfähigkeit kleiner 10 µS/cm und nachfol-gender Behandlung des Dialysats mit wässrigen Lösungen der freien Di- und/oder Tricarbonsäuren, ausgewählt aus Zitronensäure, Äpfelsäure und Weinsäure und Dialyse mit destilliertem Wasser bis das Dialysat eine elektrische Leitfähigkeit kleiner 10 µS/cm besitzt und der freie Eisenionengehalt des Dialysats kleiner 1 mg/l ist, Zusatz von N-Methyl-D-glucamin (Meglumin) und/oder 2-Amino-2-(hydroxymethyl)-1,3-propandiol (Trometamol) zur Dispersion sowie Gewinnung der eisenhaltigen Teilchen aus der hergestellten Dispersion.

### Ausführungsbeispiele

### Herstellungsbeispiel 1 (Vergleichsbeispiel):

Eisen(III)-chlorid (270 g) und Eisen(II)-chlorid(119 g) werden in 1 l dest. Wasser unter Rühren gelöst und unter Sauerstoffausschluss auf 80°C erwärmt. Durch Zugabe von Ammoniakwasser wird unter Rühren der pH-Wert der Lösung auf 10 eingestellt. Danach wird die Dispersion auf ca.60°C abgekühlt, mit Zitronensäure auf den pH 7,0 eingestellt und mit dest. Wasser dialysiert, bis das Dialysat eine elektrische Leitfähigkeit von < 10 µS/cm besitzt. Zur Entfernung größerer oder schwach aggregierter superparamagnetischer Teilchen wird die Dispersion 10 min bei 10.000 U/min zentrifugiert. Das Zentrifugat der Dispersion wird abgetrennt, in eine Ultrafiltrationsapparatur mit einem 5 kD Filter gefüllt und mit dest. Wasser so lange dialysiert, bis das Dialysat eine elektrische Leitfähigkeit von kleiner 10 µS/cm besitzt. Die Leitfähigkeit wurde mit dem Leitfähigkeitsmessgerät.der Fa. Knick bestimmt.

Das vorgereinigte Dialysat kann als Ausgangsdispersion zur Herstellung eines positiven i.v. Kontrastmittels für die MRT-Diagnostik dienen.

### Herstellungsbeispiel 2 (Vergleichsbeispiel):

Eisen(III)-chlorid (270 g) und Eisen(II)-chlorid(119 g) werden in 1 l dest. Wasser unter Rühren gelöst und unter Sauerstoffausschluss auf 80°C erwärmt. Durch Zugabe von Ammoniakwasser wird unter Rühren der pH-Wert der Lösung auf 10 eingestellt. Danach wird die Dispersion auf ca.60°C abgekühlt, mit Zitronensäure auf den pH 7,0 eingestellt und die Dispersion auf eine Leitfähigkeit von 150 mS/cm mit destilliertem Wasser eingestellt. Anschließend wird die Dispersion auf einen Magneten mit einer magnetischen Flussdichte von 0,1 T für 5 Stunden gestellt. Der Überstand der Dispersion wird abgetrennt und mit dest. Wasser dialysiert, bis das Dialysat eine elektrische Leitfähigkeit von < 10 µS/cm besitzt.

Die vorgereinigte Dispersion wird mit 50 ml einer 20 Gew. % Lösung von TriNatriumcitrat in dest. Wasser versetzt, mit dest. Wasser auf 1 I aufgefüllt und dialysiert. Dieser Vorgang wird so oft wiederholt, bis der Gehalt an freien Eisenionen kleiner als 1 mg Eisen/l ist.

### Herstellungsbeispiel 3:

Es wird eine vorgereinigte Dispersion wie in Beispiel 2 hergestellt, die eine elektrische Leitfähigkeit von < 10 µS/cm besitzt.

Die Dispersion wird mit 50 ml einer 20 Gew. % Lösung von Di-Natrium Hydrogencitrat in dest. Wasser versetzt, mit dest. Wasser auf 1 I aufgefüllt und dialysiert. Dieser Vorgang wird so oft wiederholt, bis der Gehalt an freien Eisenionen kleiner als 1 mg Eisen/l ist.

Anschließend wird die Dispersion mit einer 20 Gew. % Lösung von Citronensäure auf einen pH-Wert von 5 eingestellt und so lange mit dest. Wasser dialysiert, bis der Gehalt an freien Eisenionen kleiner als 1 mg Eisen/l ist.

### Herstellungsbeispiel 4:

100 ml der Dispersion der sehr kleinen superparamagnetischen Eindomänenteilchen von Beispiel 3, mit einem Eisengehalt von ca. 200 g Eisen/l werden mit einer Lösung von 1 molarer D(-)-N- Methylglucamin in dest. Wasser auf den pH 7,5 eingestellt. Diese Dispersion dient zu Herstellung einer galenischen Formulierung eines MR-Kontrastmittels.

### Herstellungsbeispiel 5:

In einen 100 ml Maßkolben wird eine Menge an Dispersion nach Beispiel 4 gegeben, die 2,79 g Eisen enthält. Dazu werden 6 g Mannitol und 0,304 g Natrium Glycerophosphat, gelöst in 50 ml dest. Wasser, gegeben und auf 100 ml aufgefüllt. Die entstehende galenische Formulierung wird durch einen 0,2 µm Filter in eine 100 ml Ampulle steril filtriert und die Ampulle bei 121 °C hitzesterilisiert. Die abgekühlte Dispersion kann als MRT-Kontrastmittel für die Angiographie, Lymphographie, Thromben- und Tumordiagnostik eingesetzt werden.

### Herstellungsbeispiel 6:

Eisen(III)-chlorid (270 g) und Eisen(II)-chlorid(119 g) werden in 1 l dest. Wasser unter Rühren gelöst und unter Sauerstoffausschluss auf 85°C erwärmt. Unter Zutropfen von 25%-iger Ammoniumhydroxydlösung wird ein pH-Wert von 10,5 eingestellt. Sofort nach der Fällung wird die Dispersion mit einer Lösung aus 25g Citronensäure und 25 g Weinsäure in 500 ml Wasser der pH-Wert der Dispersion auf pH 7,o eingestellt, und 20 min bei 85°C gerührt. Danach wird die Dispersion auf ca. 20°C abgekühlt, mit Salzsäure auf den pH-Wert von 7,0 eingestellt, mit 20 ml 30%-igem Wasserstoffperoxid versetzt und so lange gerührt, bis keine Gasentwicklung mehr auftritt. Die Dispersion wird 20 min mit Ultraschall von 300 W Leistung dispergiert und anschließend dialysiert, bis das Dialysat eine elektrische Leitfähigkeit von kleiner 10 µS/cm besitzt. Zur Entfernung größerer oder schwach aggregierter superparamagnetischer Teilchen wird die Dispersion 10 min bei 10.000 U/min zentrifugiert.

### Herstellungsbeispiel 7:

100 ml der vorgereinigten Dispersion der sehr kleinen superparamagnetischen Eindomänenteilchen von Beispiel 6, mit einem Eisengehalt von ca. 100 g Eisen/l, wird mit 50 ml einer 20 Gew. % Lösung von Natrium Di-Hydrogencitrat in dest. Wasser versetzt, mit dest. Wasser auf 1 I aufgefüllt und dialysiert. Dieser Vorgang wird so oft wiederholt, bis der Gehalt an freien Eisenionen kleiner als 1 mg Eisen/l ist. Anschließend wird die Dispersion mit einer 20 Gew. % Lösung von Citronensäure auf einen pH-Wert von 5 eingestellt und so lange mit dest. Wasser dialysiert, bis der Gehalt an freien Eisenionen kleiner als 1 mg Eisen/l ist.

### Herstellungsbeispiel 8:

100 ml der gereinigten Dispersion der sehr kleinen superparamagnetischen Eindomänenteilchen von Beispiel 7, mit einem Eisengehalt von ca. 100 g Eisen/l, werden mit einer Lösung von 0,1 molarer Pentaethylenhexamin in dest. Wasser auf den pH 6,0 und anschließend mit einer 1 molaren Lösung von D(-)-N- Methylglucamin in dest. Wasser auf den pH 7,5 eingestellt. Diese Dispersion dient zu Kopplung von diagnostisch wirksamen Substanzen, zell- und gewebespezifischen Bindungssubstanzen, pharmakologisch wirksamen Substanzen, pharmakologisch wirksamen Zellen oder zellfusions-vermittelnden Substanzen.

### Herstellungsbeispiel 9:

In einen 100 ml Maßkolben wird eine Menge an Dispersion nach Beispiel 8 gegeben, die 2,79 g Eisen enthält. Dazu werden 6 g Mannitol und 0,304 g Natrium Glycerophosphat, gelöst in 50 ml dest. Wasser, gegeben und auf 100 ml aufgefüllt. Die entstehende galenische Formulierung wird durch einen 0,2 µm Filter in eine 100ml Ampulle steril filtriert und die Ampulle bei 121 °C hitzesterilisiert. Die abgekühlte Dispersion kann als MRT-Kontrastmittel für die Angiographie, Lymphographie, Thromben- und Tumordiagnostik, besonders vorteilhaft für die Diagnostik von Prostata-Tumoren eingesetzt werden.

### Herstellungsbeispiel 10:

Eine Lösung von 65 mg Fluoresczeinisothiocyanat in 10 ml DMF wird mit einer Lösung vom 950 mg Pentaethylenhexamin in 10 ml DMF gemischt. Es fällt ein orangefarbiger Niederschlag aus. Nach einer Stunde wird der Niederschlag in Wasser gelöst und zu 100 ml der Dispersion der sehr kleinen superparamagnetischen Eindomänenteilchen von Beispiel 7, mit einem Eisengehalt von ca. 100 g Eisen/l, gegeben. Anschließend wird die Dispersion mit einer 1 molaren Lösung von Trometamol (2-Amino-2-(hydroxymethyl)-1,3-propandiol) in dest. Wasser auf den pH 7,5 eingestellt. Die Mischung wird in eine Ultrafiltrationsapparatur mit einem 50 kD Filter gefüllt und mit dest. Wasser so lange dialysiert, bis das Dialysat eine elektrische Leitfähigkeit von kleiner 10 µS/cm besitzt. Die gereinigte Dispersion kann zur Herstellung eines MRT-Kontrastmittel für die Angiographie, Thromben- und Tumordiagnostik, besonders vorteilhaft für die Diagnostik der Sentinel-Lymphknoten bei Mamacarzinomen, sowie zum Labeling von lebenden Zellen, eingesetzt werden.

Typische Analysendaten der sehr kleinen superparamagnetischen Eindomänenteilchen sind:

| | |
|---|---|
| Teilchendurchmesser d50 | 3,8 nm |
| Gesamtdurchmesser | |
| mit Stabilisator | : 9 nm |
| T1-Relaxivität | : 20 l/mmol s |
| T2-Relaxivität | : 38 l/mmol s |
| Verhältnis der Relaxivitäten R2/R1 | : 1,9 |

### 1) Einfluss der Herstellung, der Reinigung, der Formulierung und der Hitzesterilisation auf die Verträglichkeit an Ratten nach intravenöser Injektion der Proben

Die Verträglichkeit wurde an Ratten untersucht (männlich, Wistar, 200-250g). Als Parameter diente das Non-Toxic-Dose-Level. Das ist die Dosis bei der keine Ratte einer Versuchsgruppe (n=3 Tiere je Dosisgruppe) innerhalb von zwei Wochen nach intravenöser Injektion einer Probe gestorben ist.

In einem weiteren Versuch wurde der Einfluss der intravenösen Injektion einer Probe auf die Proteinausscheidung und die Hämoglobinausscheidung (Hämolyse) untersucht. Hierzu wurden die Ratten nach intravenöser Injektion der Probe in eine mit dest. Wasser gereinigte Kunstoffbox gesetzt und der Abgang von Spontanurin beobachtet. Es wurde stündlich bis vier Stunden nach Injektion der Spontanurin untersucht. Der Spontanurin wurde innerhalb weniger Sekunden nach Abgang gesammelt und auf einen Urin-Teststreifen (Combistix^{R}, Bayer AG) aufgebracht. Das Proteinfeld und das Hämolysefeld wurden innerhalb der vom Hersteller vorgegebenen Zeit abgelesen.

Die Ergebnisse der Untersuchungen sind in Tabelle1 dargestellt. Es zeigt sich, dass die nach Beispiel 1 (WO 97/35200) hergestellte stabilisierte und vorgereinigte Probe, die lediglich mit 6 % Mannitol versetzt wurde, nach Hitzesterilisieren,extrem unverträglich ist und die Tiere bereits bei geringsten Dosierungen sterben, so dass eine Messung der Nierenphysiologie nicht möglich ist.

Eine Verringerung der Toxizität wird durch die Behandlung der wässrigen Dispersion von mit Zitronensäure stabilisierten und vorgereinigten superparamagnetischen Eindomänenteilchen mit Lösungen von Tri-, Di-, und Mono-Salzen der Zitronensäure und anschließender Dialyse mit destilliertem Wasser erreicht (vgl. Beispiel 2).

Eine weitere Verbesserung der Verträglichkeit erreicht man durch die erfindungsgemäße Reinigung mit Zitronensäuresalzen gemäß Beispiel 2 und Einstellen eines pH-Wertes von ca. 5 mit freier Zitronensäure und anschließender Dialyse mit destilliertem Wasser (vgl. Beispiel 3). Durch Neutralisation der so entstandenen Dispersion mit einer Lösung von D(-)-N- Methylglucamin in destilliertem Wasser wird eine weitere Verringerung der Toxizität erreicht (vgl. Beispiel 4).

Durch die Formulierung der galenischen Zubereitung mit dem Komplexbildner Natrium-Glycerophosphat (vgl. Beispiel 5) werden Dosierungen bis 3 mmol Fe/kg ohne Nebenwirkungen (Proteinurie, Hämolyse) sehr gut als Bolusinjektion von den Ratten vertragen.

**Tabelle 1: Einfluss von Reinigung und galenischer Formulierung auf die Verträglichkeit der Proben nach intravenöser Injektion an Ratten**

| | Beispiel 1 | Beispiel 2 | Beispiel 4 | Beispiel 5 |
|---|---|---|---|---|
| | vorgereinigt | gereinigt Na-Citrat | gereinigt nach Beispiel 3 Methylglucamin | gereinigt nach Beispiel 4 mit Na-Glycerophosphat |
| | unformuliert | unformuliert | unformuliert | formuliert |
| | hitzesterilisiert | hitzesterilisiert | hitzesterilisiert | hitzesterilisiert |
| Non-Toxic-Dose-Level | > 0.1 mmol Fe/kg kein Urin gewinnbar da toxische Dosis sehr | bis 1 mmol Fe/kg | bis 2 mmol Fe/kg | bis 3 mmol Felkg |
| Hämoglobinurie | niedrig | ab 0.5 mmol Fe/kg | ab 1,5 mmol Fe/kg | ab 2 mmol Fe/kg |
| | kein Urin gewinnbar da toxische Dosis sehr | | | |
| Proteinurie | niedrig | ab 0.5 mmol Fe/kg | ab 1,5 mmol Felkq | ab 2 mmol Fe/kg |

Non-Toxic-Dose-Level: Dosis, bei der keine Ratte einer Versuchsgruppe innerhalb von zwei Wochen nach Injektion gestorben ist

Die Ergebnisse an Ratten zeigen, dass durch die Reinigungsschritte und die galenischen Zusätze ein biologisch anwendbares Produkt erreicht wird, dass nach intravenöser Injektion auch bei sehr hohen Dosierungen, die bis ca. 100-fach über einer klinisch notwendigen Dosis liegen, keine toxischen Effekte auftreten.

### 2) Einfluss der Herstellung, der Reinigung, der Formulierung und der Hitzesterilisation auf die Wirksamkeit an Ratten nach intravenöser Injektion der Proben.

An männlichen Wistar-Ratten (200-250 g) wurde die Zirkulationsdauer von Proben der Beispiele 1, 2 und 5 im Blut mittels der Messung der magnetischen Wirkung bestimmt. Hierzu wurden die Proben in einer Dosis von 0.05 mmol Fe/kg intravenös injiziert. Es erfolgten Blutabnahmen vor und 1, 2, 5, 10, 15, 20, 30, 60, 90, 180 und 240 min nach Injektion der Proben. Es wurde die Relaxationszeit (longitudinale und transversale Relaxationszeit) im Blut gemessen mittels Relaxometrie bei 0.94 T (Bruker Minispec, Bruker, Karlsruhe). Anhand der Zeit (nach Injektion) und der Relaxationszeiten im Blut wurde ein Wirkungs-Zeit-Verlauf ermittelt.

Durch die Anpassung dieser Daten an eine einfache exponentielle Funktion wurde die Bluthalbwertszeit berechnet. Die Bluthalbwertszeit ist ein pharmakokinetischer Parameter, der die Klärung des Wirkstoffes aus dem Blut beschreibt. Je länger der Wirkstoff im Blut zirkuliert, um so größer ist die Bluthalbwertszeit.

**Tabelle 2: Einfluss von Reinigung und galenischer Formulierung auf die Wirksamkeit der Proben nach intravenöser Injektion an Ratten**

| | Beispiel 1 | Beispiel 2 | Beispiel 4 | Beispiel 5 |
|---|---|---|---|---|
| | vorgereinigt | gereinigt Na-Citrat | gereinigt nach Beispiel 3 Methylglucamin | gereinigt nach Beispiel 4 mit Na-Glycerophosphat |
| | unformuliert | unformuliert | unformuliert | formuliert |
| | hitzesterilisiert | hitzesterilisiert | hitzesterilisiert | hitzesterilisiert |
| Bluthalbwertszeit | 5 min | 10 min | 20 min | 40 min |

Überraschenderweise zeigen die Ergebnisse, dass durch die erfindungsgemäße Reinigung und die Formulierung mit Natrium-Glycerophosphat auch die Verweildauer der Partikel im Blut verlängert wurde. Dies steht im Gegensatz zur Verträglichkeit, da man eher vermutet, dass Partikel, welche lange im Blut verweilen und nicht so schnell herausgefiltert werden, eine toxische Wirkung entfalten können.

Für die Anwendung als Diagnostikum oder für die Therapie ist eine lange Bluthalbwertszeit von Vorteil, da auf diese Weise eine höhere Konzentration im Zielgewebe erreicht werden kann, bevor die Partikel aus dem Blut geklärt sind.

Durch die ausreichende Verträglichkeit der Partikel und die lange Zirkulation im Blut können die erfindungsgemäßen Formulierungen vorteilhaft in der medizinischen und Diagnostik und Therapie eingesetzt werden.

### Anwendungsbeispiel 1: Gefäßdiagnostik

Die Partikel können als positives (hellmachend) und negatives (dunkelmachend) Kontrastmittel für die magnetresonanztomographische Beurteilung des Lumens, der Wand und der morphologischen Charakterisierung von Einengungen oder Verschlüssen von Gefäßen (Arterien, Venen) des Körperstammes, der Extremitäten, der Kopf-Halsregion einschließlich intrakranieller Gefäße, von herznahen Gefäßen und Herzkranzgefäßen Anwendung finden. Dies ist nach intravenöser und intraarterieller Bolusinjektion möglich.

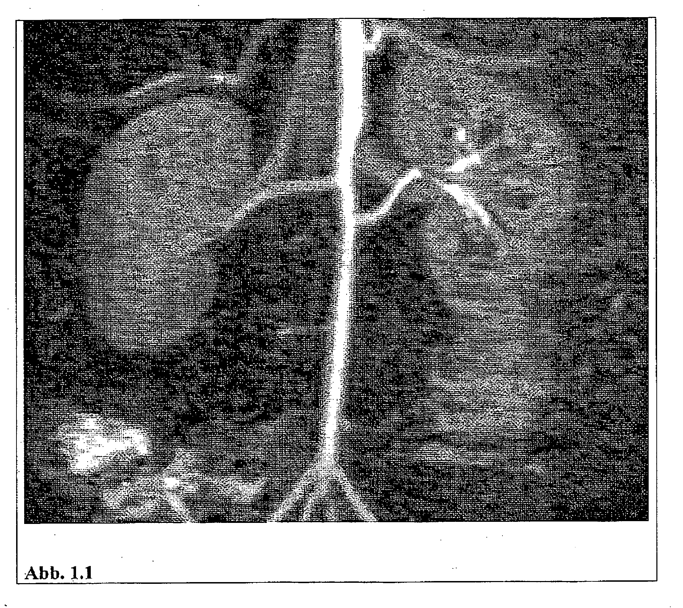

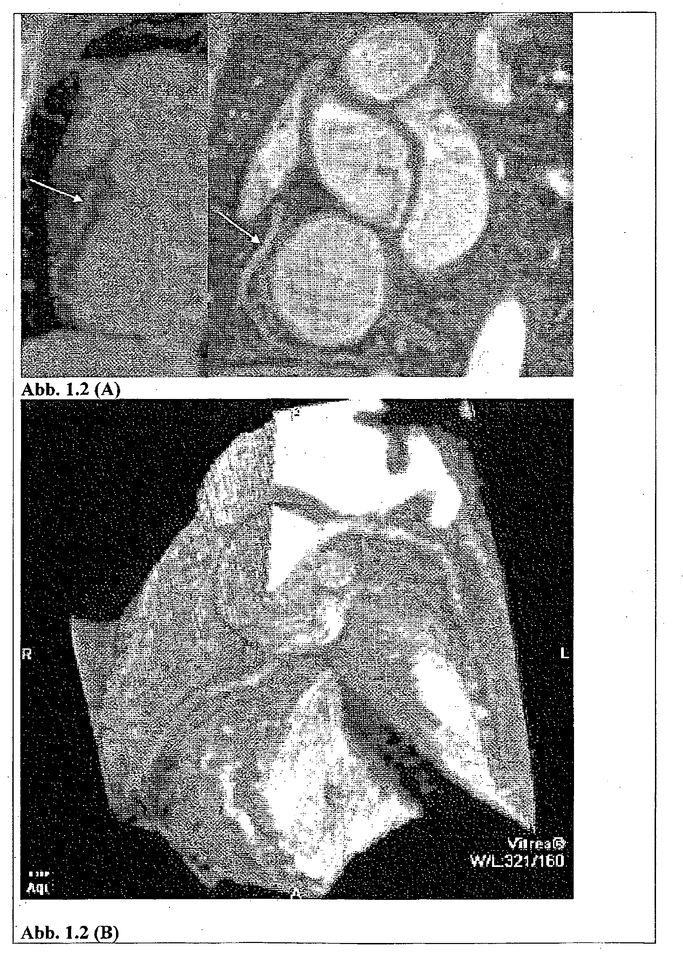

Anhand der Bildbeispiele ist die Bolusangiographie am Schwein (Abbildung 1.1) und eine Equilibrium-Angiographie der Herzkranzgefäße eines gesunden Probanden (Abbildung 1.2) dargestellt.

Die gute Verträglichkeit der Partikel ermöglicht auch eine Bolusinjektion am Menschen. Die lange Zirkulationsdauer ermöglicht eine hochaufgelöste Darstellung der Herzkranzgefäße am Menschen (Abbildung 1.2).

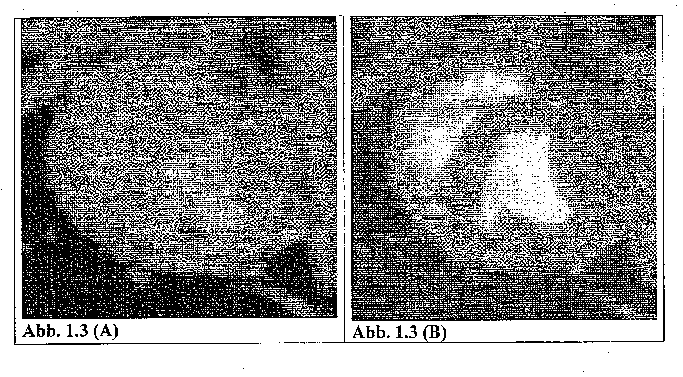

Neben der Beurteilung der großen Gefäße ermöglichen die Eisenoxid-Nanopartikel der Erfindung eine Beurteilung der Mikrozirkulation einschließlich der Angiogenes im Rahmen von Entzündungsprozessen, Infekten oder Tumoren. Als Beispiel ist in Abbildung 1.3 die Myokardperfusion an einem Schwein dargestellt mit einer Minderperfusion in einem künstlich erzeugten Herzinfarkt (Abbildung 1.3)

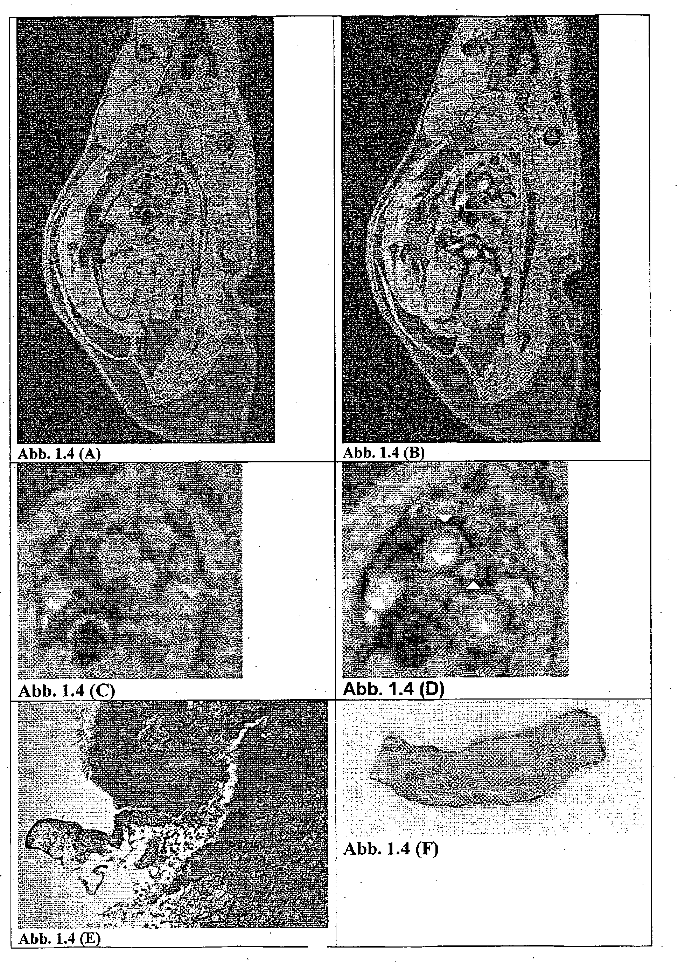

Durch die Anreicherung der erfindungsgemäßen Partikel in entzündlich veränderten Arterienwänden (verschiedene Stadien der Arteriosklerose) ist es möglich eine Gefährdung für einen Infarkt unabhängig von dem Ausmaß einer Gefäßeinengung sehr früh zu erkennen. Dies ist in Bildbeispiel Abbildung 1.4 dargelegt. Des weiteren können die Partikel zur morphologischen Beurteilung von Thromben oder Emboli (arteriell oder venös) Anwendung finden.

### Anwendungsbeispiel 2: Diagnostik von Tumoren und deren Metastasen einschließlich Metastasierungswege des Lymphsystems

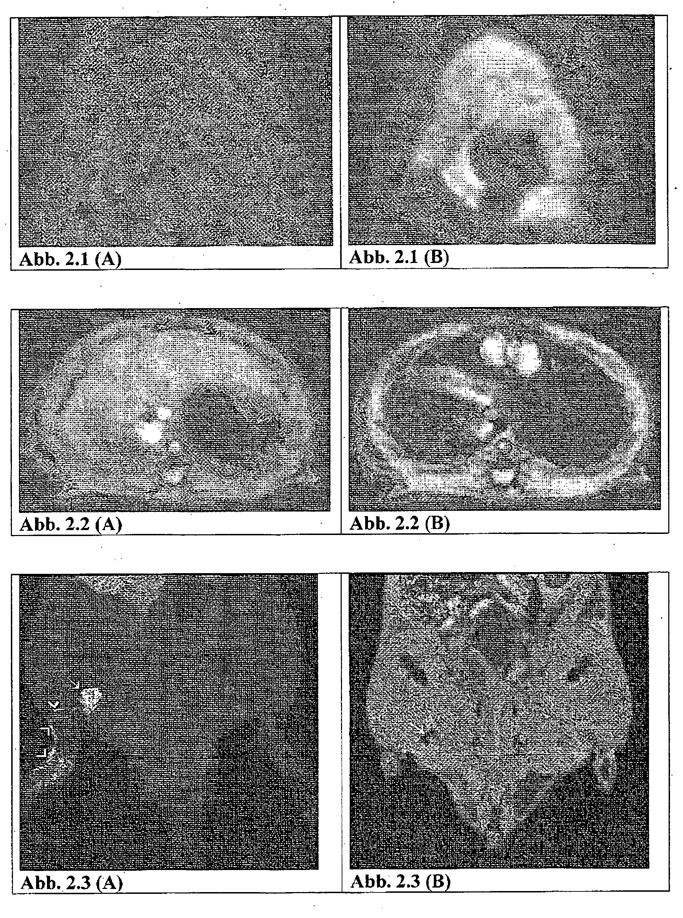

Die gut verträglichen gereinigten und formulierten Eisenoxid-Partikel des Beispiels 5 werden zur MRT-Diagnostik von Primärtumoren und deren Metastasen verwendet. Dies ist mit T1-gewichteter Bildgebung (hellmachende Wirkung, Abbildung 2.1) und T2-gewichteter (dunkelmachende Wirkung Abbildung 2.2) möglich. In Bildbeispiel Abbildung 2.1 ist die Anwendung für die verbesserte Darstellung eines Lebertumors an der Ratte gezeigt.

Die magnetischen Eigenschaften und die gute Verträglichkeit der gereinigten und formulierten Partikel der Erfindung ermöglichen eine Injektion in das lymphatische System, oder in Regionen (Körpergewebe, Organe Tumoren) von denen Gewebswasser (Lymphe) zu einem Lymphknoten hintransportiert wird. Bei der Tumordiagnostik dient dies zur Auffindung des Sentinellymphknotens oder auch Wächterlymphknotens. Dies ist der entscheidende Lymphknoten, der als möglicher erster Lymphknoten Metastasen durch einen Primärtumor erhält. Ein möglicher metastatischer Befall des Sentinellymphknotens entscheidet über die Therapieplanung und die Prognose bei Tumorerkrankungen. Durch Injektion der Eisenoxid-Nanoteilchen des Beispiels 5 in die Primärtumorregion kann mit T1-gewichteter Bildgebung das Lymphgefäßsystem beurteilt werden (hellmachende magnetische Wirkung der Eisenoxid-Nanoteilchen). Mit T2-gewichteter MRT-Bildgebung kann der Lymphknoten und eventuelle Metastasen beurteilt werden (dunkelmachende magnetische Wirkung der Eisenoxid-Nanoteilchen). Dies ist in den Bildbeispielen 2.3 dargestellt. Die Beurteilung des Lymphsystems kann durch zusätzliche Bindung von Farbstoffen (visuell, fluoreszenztechnisch) oder durch die Bindung von radioaktiven Stoffen (Technetium, Indium) zu einer MRT-optischen Bildgebung oder zu einer MRT-Nuklearmedizinischen Bildgebung kombiniert werden.

### Anwendungsbeispiel 3: Tumortherapie

Die sehr gute Verträglichkeit und die lange Zirkulationsdauer der gereinigten und formulierten Eisenoxid-Partikel der Erfindung ermöglicht eine Anwendung für die Tumortherapie nach intravenöser, intraarterieller und intratumoraler Injektion in Kombination mit Magnetfeldern (Magnetfeldhyperthermie), Embolisaten und Chemotherapeutika. Dadurch kann eine erhöhte Anreicherung im Zielgewebe durch Bindung sogenannter targetspezifischer Liganden auf den Eisenoxid-Partikeln erreicht werden. In Beispiel Abb. 3.1 ist die Verstärkung der intratumoralen Anreicherung der Partikel durch die Bindung von Polyaminen auf der Oberfläche und damit die Zielrichtung an angioge-netisches Endothel dargestellt. Daneben ermöglichen die unmodifizierten Partikel durch die lange intravasale Verweildauer eine Erfassung der Mirkozirkulation von Tumoren und damit möglicher Therapieeffekte im Rahmen der Tumortherapie (Therapiemonitoring).

### Anwendungsbeispiel 4: In-Vivo Zellmonitoring

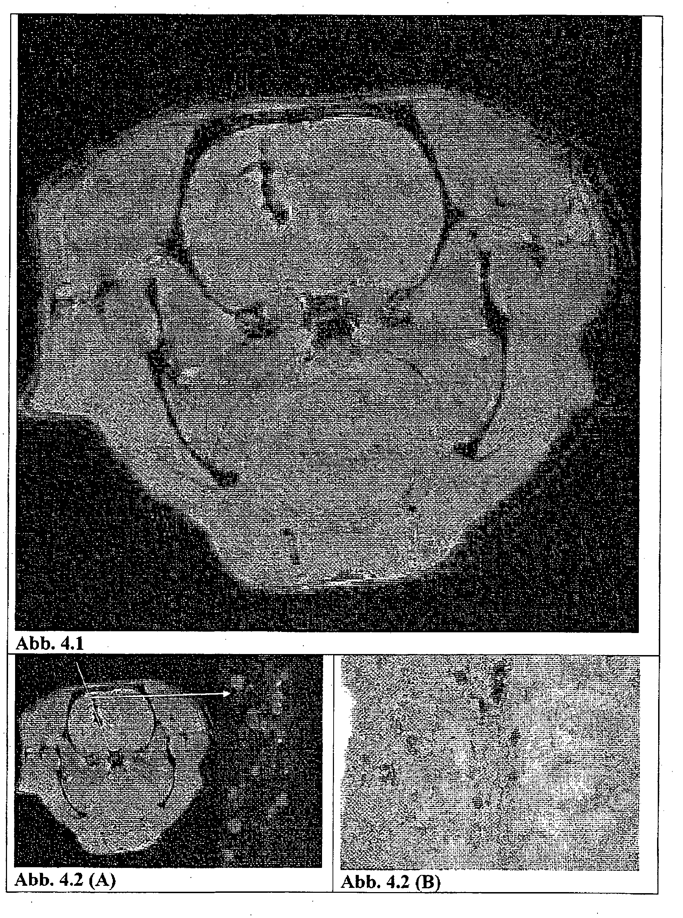

Mit den gut verträglichen gereinigten und formulierten Eisenoxid-Partikeln können Zellen (Stammzellen, Endothelzellen, dendritische Zellen, Organzellen, Immunzellen) außerhalb des Körpers markiert werden, indem die Zellen mit z. B. mit einer Dispersion nach Beispiel 3 für 30 bis 60 min inkubiert und gewaschen werden. Nach Injektion dieser markierten Zellen in den Körper (intravenös, intraarteriell, lymphatisch, in Gewebe, Organe oder pathologische Prozesse) ist es möglich, diese Zellen innerhalb des

lebenden Körpers zu verfolgen. Als Beispiel ist die Darstellung der Markierung von neuronalen Stammzellen aufgezeigt an einem Parkinson-Modell an der Ratte (Abb. 4.1 und 4.2).

Durch die Bindung von Farbstoffen oder radioaktiven Markern ist eine Kombination von MRT und optischer Bildgebung oder eine Kombination von MRT und Nuklearmedizinischer Bildgebung, wie SZINTIGRAPHIE, SPECT oder PET, möglich, um die Morphologie, Funktion und Biochemie von mit superparamagnetischen Teilchen markierten Zellen im lebenden Organismus zu untersuchen.

### Rezeptorimaging für die Diagnostik entzündlicher Prozesse

Bei entzündlichen Prozessen im menschlichen Körper findet eine Anhäufung von körpereigenen Immunzellen, wie z. B. Makrophagen, statt. Diese Makrophagen nehmen die superparamagnetische Nanopartikel auf und es kommt zu einer Anreicherung der erfindungsgemäßen Partikel des Beispiels 5 in den Entzündungsregionen. Diese "magnetisierbaren Makrophagen" können im MR-Tomographen bei T2-gewichteten Aufnahmen sichtbar gemacht werden. Anwendungsgebiete sind z. B. der Rheumatismus.

### Arteriosklerose

Die Anreicherung der magnetischen Partikel aus der Probe des Beispiels 5 in dem arteriosklerotischen Plaque führt zu einer T2-Relaxationszeit verkürzenden Wirkung mit einem Signalverlust in der Gefäßwand. Die arteriosklerotischen Plaque werden dunkel kontrastiert dargestellt. Die Anreicherung der magnetischen Partikel dieser Probe zeigt das Vorliegen von Entzündungszellen in und Makrophagen in dem arteriosklerotischen Plaque.

### MRT neurodegenerativer Krankheiten

Bei vielen neurodegenerativen Erkrankungen wie Morbus Alzheimer oder Multipler Sklerose spielt eine erhöhte Apoptose eine herausragende Bedeutung. Erste Versuche mit sehr kleinen Teilchen des Beispiels 4 (Teilchendurchmesser 3,5 nm) zeigen bei Mäusen mit passiver experimenteller autoimmuner Enzephalomyelitis (EAE) eine Anreicherung der Teilchen im durch Multiple Sklerose (MS) veränderten Bereich der Großhirnrind.

Durch die Bindung von Farbstoffen oder radioaktiven Markern können MR-Kontrastmittel für die parenterale Anwendung hergestellt werden, die eine Kombination von MRT und optischer Bildgebung oder eine Kombination von MRT und Nuklearmedizinischer Bildgebung, wie SZINTIGRAPHIE, SPECT oder PET, ermöglichen.

Überwachung therapiebedingter Apoptose z. B. bei der Tumortherapie von Annexin V gekoppelten erfindungsgemäßen Teilchen

Bisher wurde der Erfolg einer Tumortherapie vorwiegend anhand morphologischer Kriterien eingeschätzt, die jedoch in der Regel erst nach Wochen oder gar Monaten zu erfassen sind. Die in vivo Bildgebung von Apoptose kann dagegen eine frühe bzw. simultane Überwachung des Therapieerfolgs ermöglichen, da eine der resultierenden Tumorregression vorausgehende Apoptose-Induktion schon innerhalb von Stunden bzw. weniger Tage eingeleitet wird. Die in vivo Bildgebung dieser frühen Veränderungen kann die notwendige Zeit zur Einschätzung eines Therapieerfolges stark verkürzen, was es letztendlich ermöglicht, bei Bedarf Therapiekonzepte wesentlich früher zu ändern. Dadurch ist es möglich, zur Erhöhung der Behandlungschancen wichtige Zeit zu gewinnen und unnötige Nebenwirkungen, aber auch Kosten einer unwirksamen Therapie zu reduzieren. Idealer Weise kann die Apoptose-Bildgebung Echtzeit-Informationen über die räumliche Verteilung von Apoptose liefern und damit eine aufschlussreiche Charakterisierung pathologischer Prozesse unterschiedlichster Krankheitszustände erlauben.

### Thrombosenachweis durch MRT

Mit den erfindungsgemäßen Teilchen können akute Thromben, wie durch Untersuchungen an Ratten und Kaninchen gezeigt werden konnte, durch MRT nachgewiesen werden.

### Therapie entzündlicher Plaques

Die Anreicherung der magnetischen Partikel aus der Probe des Beispiels 5 in dem arteriosklerotischen Plaque führt zu einer T2-Relaxationszeit verkürzenden Wirkung mit einem Signalverlust in der Gefäßwand. Die arteriosklerotischen Plaque werden dunkel kontrastiert dargestellt. Die Kopplung von entzündungshemmenden Substanzen an die erfindungsgemäßen Teilchen, wie Paclitaxel oder Matrix-Metallo-Proteinasehemmern (MMP), wie z. B. Marimastat, Neovastat, Sirolimus oder Tacrolimus, führt zu einer Anreicherung dieser entzündungshemmenden Substanzen in den entzündlichen Plaques und damit zu einer Entzündungshemmung.

### Transfektionsvehikel in der Gentherapie

Experimentelle wurde gefunden, dass erfindungsgemäße Teilchen mit polyaminbeschichteten Oberflächen nach Beispiel 8 als Transfektionsmittel für DNA und RNA in vitro an Zellkulturen von Colonkarzinomen geeignet sind. Die Anreicherung der DNA und RNA , gebunden an die magnetischen Partikel, führt in den Zellen zu einer T2-Relaxa-tionszeit verkürzenden Wirkung mit einem Signalverlust in den Zellen und kann so als Maß des Transfektionserfolges dienen.

### Adjuvanz zur Immunsteigerung gegen Viren, Bakterien und Tumorzellen

Therapieversuche mit Konjugaten der erfindungsgemäßen Teilchen nach Beispiel 8 mit Zellwandbestandteilen von Tumorzellen wurden im Tierversuch an implantierten Prostata- und Colonkarzinomen durchgeführt. Eine Immunreaktion wurde beobachtet, die zur Nekrose des Tumors führte.

Es zeigen die Abbildungen:
- Abbildung 1.1:: Magnetresonanztomographische Darstellung der Nierenarterien am Schwein während Bolusinjektion der Probe des Herstellungsbeispiels 5 (arterielle MRT-Bolusangiographie). MRT-Bolusangiographie am Schwein der Nierenarterien und der Aorta bei 1.5 Tesla mit einer T1-gewichteten Gradienten-Echo-Technik Repetitionszeit 6 ms Echozeit 1.7 ms Anregungswinkel 25°. Es wurde eine Dosis von 0.025 mmol Fe/kg der Probe des Herstellungsbeispiels 5 intravenös als schneller Bolus injiziert. Durch die Anflutung des Kontrastmittels in den arteriellen Gefäßen erhält man eine angiographische Abbildung der Gefäße ohne die Darstellung von Venen. Die Bolusinjektion wird durch die gute Verträglichkeit der Probe des Beispiels 5 erst möglich. Die hohe magnetische Wirksamkeit führt zu einer starken Verkürzung der T1-Relaxivität des Blutes dies führt zu einer hellen Darstellung der Gefäße.
- Abbildung 1.2:: Magnetresonanztomographische Darstellung der Herzkranzgefäße (MRT-Koronarangiographie) am Menschen nach Injektion der Probe des Herstellungsbeispiels 5. Durch die Injektion der Probe konnte am Menschen die Darstellung der Herzkranzgefäße verbessert werden. Untersucht wurde eine gesunder Proband bei 1.5 Tesla mit einer Gradienten-Echo-Technik mit einer Repetitionszeit von 4.5 ms, einer Echozeit von 1.7 ms und einen Anregungswinkel von 25° vor und nach Injektion der Probe in einer Dosis von 0.045 mmol Fe/kg. Ein Abschnitt der rechten Herzkranzarterie an einem gesunden Probanden ist mit einem Pfeil markiert. Vor Kontrastmittelgabe ((A)linke Bildseite) ist der dargestellte Abschnitt der rechten Herzkranzarterie unscharf und unterbrochen dargestellt. Mit der erfindungsgemäßen Kontrastmittel-probe ist der Gefäßabschnitt scharf und kontrastreich zu sehen. Ebenso stellen sich die Herzkammern sehr hell dar, was eine gute Abgrenzung zur Herzmuskulatur erlaubt. Der Kontrast bleibt nach Injektion über einen Zeitraum von bis zu 50 Minuten erhalten. Dies erlaubt die hoch aufgelöste Darstellung des gesamten Gefäßsystems des Herzen. In Abbildung 1.2 (B) wurde aus den gemessenen Einzelschichten eine dreidimensionale Rekonstruktion der Herzkranzgefäße durchgeführt. Dies ist nur durch die hohe Wirksamkeit und die lange Verweildauer im Blut der erfindungsgemäßen Probe möglich.
- Abbildung 1.3:: Mikrozirkulation im gesunden Herzgewebe im Vergleich zum Infarkt am Schwein in der Equilibriumphase. Darstellung der Mikrozirkulation am Beispiel eines künstlich erzeugten Infarktes am Schwein. Die MRT-Untersuchung erfolgte bei 1.5 Tesla mit einer elektrokardiographisch getriggerten Gradienten-Echo Technik mit einer Repetitionszeit von 5 ms, einer Echozeit von 2 ms und einem Anregungswinkel von 25 °. Es ist eine Untersuchung während der Equilibriumsphase. Da die Probe des Herstellungsbeispiels 5 lange im Blut verweilt und dort die magnetische Kontrastmittelwirkung entfaltet ist das gut durchblutete Herzmuskelgewebe hell dargestellt, im Vergleich zu dem dunkel schlecht durchbluteten Infarktareal (B) im unteren Rand des kreisförmig dargestellten linken Herzmuskels. Eine sichere Abgrenzung des Infarktes ist ohne Kontrastmittel nicht möglich (A).
- Abbildung 1.4:: Darstellung der Gefäßwandmorphologie mit Entzündung an einem Model für Arteriosklerose am Watanabe-Kaninchen mit erblicher Hyperlipidämie (Doppelkontrast-MRT-Angiographie am Kaninchen zur Darstellung arteriosklerotischer Plaque). Untersucht wurden Kaninchen an einem klinischen MR-Tomographen bei 1.5 Tesla mit einer mäßig T1-gewichteten Gradienten-Echo Technik mit einer Repetitionszeit von 100 ms, einer Echozeit von 3.2 ms und einem Anregungswinkel von 25°. Vor intravenöser Injektion der Probe des Herstellungsbeispiels 5 sind die zentralen Halsgefäße dunkel dargestellt, wobei die Gefäßwand heller erscheint (A und vergrößerter Ausschnitt C). Nach Injektion der Probe wird das Lumen der Gefäße heller, also signalreicher, dargestellt durch die T1-Relaxationszeit verkürzende Wirkung der Probe (B und vergrößerter Ausschnitt D). Die hellen weißen Pfeilköpfe in D zeigen die Halsgefäße. Die Anreicherung der magnetischen Partikel aus der Probe in dem arteriosklerotischen Plaque führt zu einer T2-Relaxationszeit verkürzenden Wirkung mit einem Signalverlust in der Gefäßwand. Die arteriosklerotischen Plaques werden dunkel kontrastiert dargestellt. Die Anreicherung der magnetischen Partikel der Probe zeigt das Vorliegen von Entzündungszellen und Makrophagen in dem arteriosklerotischen Plaque. Durch die magnetischen Eigenschaften der Partikel von der Probe ist die Untersuchung von Gefäßen mit einem doppelten Kontrast möglich. Das gesunde Gefäßlumen wird durch die frei zirkulierenden Partikel hell dargestellt und die gefährlichen Gefäßwandveränderungen werden dunkel bzw. geschwärzt dargestellt. Makroskopisch im Sektionspräparat (F) und im histologischen Schnitt (E) der Aorta von diesem Kaninchen kann die Anreicherung der eisenhaltigen magnetischen Partikel durch die Berliner-Blau-Eisenreaktion (angereichertes Eisen ist blau) dargestellt werden. Histologisch erkennt man die Anreicherung der magnetischen Partikel der Probe in den Makrophagen des arteriosklerotischen Plaque (E). Die Makrophagen stehen für entzündliche und damit gefährliche Veränderungen in der Gefäßwand, welche zu einem plötzlichen Herzinfarkt führen können, da an dieser Stelle die Gefäßwand reißen kann, was zur Bildung eines Thrombus führt.
- Abbildung 2.1:: Magnetresonanztomographische Darstellung eines Lebertumors (implantiertes Colon-Carcinom CC531) an der Ratte in T1-gewichteter Bildgebung mit positivem Kontrast. Untersuchung in frontaler Schicht-orientierung bei 1.5 Tesla mit einer T1-gewichteten Gradienten-Echo Sequenz mit einer Repetitionszeit von 6.8 ms, einer Echozeit von 2.3 ms und einem Anregungswinkel von 25°. Vor Kontrastmittelinjektion (A) ist der Tumor in der Leber nur schwer abgrenzbar. Nach Injektion der Probe des Herstellungsbeispiels 5 in einer Dosis von 0,03 mmol Fe/kg KGW (B) ist die Leber sehr hell und signalreich dargestellt. Der dunkle Tumor am unteren Rand grenzt sich deutlich ab. Man erkennt sehr genau den oberen großen Anteil des Tumors und den unteren kleinen Teil.
- Abbildung 2.2:: Magnetresonanztomographische Darstellung eines Lebertumors (implantiertes Colon-Carcinom CC531) an der Ratte in T2-gewichteter Bildgebung mit negativem Kontrast. Untersuchung in axialer Schichtorientierung bei 1.5 Tesla mit einer T2-gewichteten Gradienten-Echo Sequenz mit einer Repetitionszeit von 200 ms, einer Echozeit von 12 ms und einem Anregungswinkel von 12°. Vor Kontrastmittelinjektion (A) ist die Leber sehr hell und der Tumor in der Leber ist nicht abgrenzbar. Der mit Nahrung und Luft gefüllte Magen (rechts in Bild A) ist dunkel dargestellt Nach Injektion der Probe des Herstellungsbeispiels 5 in einer Dosis von 0,03 mmol Fe/kg KGW (B) wird die Leber durch die T2-Relaxationszeit verkürzende Wirkung der magnetischen Teilchen schwarz dargestellt. Nun erkennt man den signalreichen (hellen) Tumor am oberen Rand der Leber sehr gut.
- Abbildung 2.3:: Magnetresonanztomographische Lymphographie und Lymph-angiographie an der Ratte in T1 und T2-gewichteter Bildgebung. Die Untersuchungen erfolgten bei 1.5 Tesla in frontaler Schichtorientierung. Es wurden 0.02 ml einer Lösung mit 0.02 mmol Eisen pro ml der Probe des Beispiels 5 in die rechte hintere Pfote der Ratte injiziert. In der T1-gewichteteten Messtechnik (A) mit einer Gradienten-Echo Technik mit einer Repetitionszeit von 50 ms, einer Echozeit von 5 ms und einem Anregungswinkel von 25° erkennt man die Lymphgefäße, welche die Lymphe vom Injektionsort (Pfote) zu dem Sentinellymphknoten (hell dargestellt) transportiert (kleine Pfeilköpfe). Der kleine Pfeil deutet auf den Lymphknoten. Hier ist die helle Lymphe in den Randsinus des Lymphknotens dargestellt. Die Untersuchung erfolgte ca. 5 min nach Injektion. In der T2-gewichteteten Gradienten-Echo-Messung (B) mit einer Repetitionszeit von 100 ms, einer Echozeit von 11 ms und einem Anregungswinkel von 15° sieht man den eigentlichen Lymphknoten, in dem sich die Eisenpartikel der Probe angereichert haben. Die Anreicherung führt in dieser Messtechnik zu einer Signalauslöschung (Pfeil).
- Abbildung 3.1:: Magnetresonanztomographische Darstellung des Targeting der Angiogenese mit den mit Polyamin modifizierten Eindomänenteilchen der Probe des Herstellungsbeispiels 8 an dem Prostata-Carcinom Dunning Tumor G-Zell-Linie der Ratte. Die Untersuchung der Ratten erfolgte bei 1.5 Tesla in axialer Schichtorientierung mit einer T2-gewichteten Gradienten-Echo Technik (Repetitionszeit 200 ms, Echozeit 15 ms, Anregungswinkel 15 °). (A) und (C) sind Untersuchungen vor Injektion der Proben. Der Tumor ist im Vergleich zum umliegenden Gewebe heller dargestellt. Nach intravenöser Injektion der Probe des Herstellungsbeispiels 5 (B) in einer Dosis von 0.045 mmol Fe/kg Körpergewicht, erkennt man nach Injektion, dass Tumorareale inhomogen heller und auch dunkler werden, im Vergleich zu der Aufnahme vor intravenöser Injektion der Probe (A). Dies zeigt Areale mit einer hohen Gefäßdichte (hellere Areale) und Areale mit einer Anreicherung im Tumorgewebe (dunklere Areale). Abbildung 3.1 (D) zeigt nach intravenöser Injektion der Probe des Beispiels 8 in einer Dosis von 0.045 mmol Fe/kg eine starke Anreicherung dieser Partikel im Tumor (dunkel). Das angiogenetische Gefäßendothel hat eine hohe Dichte an Rezeptoren für positive Ladungen. Durch die Modifikation mit Amin wird die Oberfläche der Partikel positiv. Dadurch zeigt sich im Vergleich zu der negativen Probe des Beispiels 5 eine sehr starke Anreicherung der Partikel im Tumorgewebe, die zu einer dramatischen Signalreduktion (Schwärzung, (D)) führt, im Vergleich zum Leerbild (C).
- Abbildung 4.1:: Magnetresonanztomographisches Monitoring von zuvor mit Eisenoxidpartikeln gelabelten und anschließend implantierten neuronalen Stammzellen im Gehirn von Ratten. Die Untersuchungen wurden bei 7 Tesla durchgeführt mit einer T2-gewichteten Gradienten-Echo-Technik (Repetitionszeit 490 ms, Echozeit 5.4 und Anregungswinkel 15°). MR-tomographische Darstellung einer Ratte 16 Wochen nach Implantation von 100.000 neuronalen Stammzellen, die vor der Implantation mit der Probe des Beispiels 5 inkubiert wurden. Im Gehirn erkennt man einen Bereich mit Signalauslöschung (schwarz) durch die Zellen, welche mit der Probe markiert wurden. Die Zellen können noch nach 16 Wochen an der Implantationsstelle MR-tomographisch dargestellt werden
- Abbildung 4.2:: Magnetresonanztomographisches Monitoring von implantierten neuronalen Stammzellen im Gehirn von Ratten im Vergleich zu Fluoreszenzmarkierung (A) und Eisenfärbung (B). Von dem Gehirn der Ratte wurden nach Ende der MRT-Untersuchung histologische Schnitte angefertigt in einer Orientierung entlang des Stichkanales (A, weißer Strich). Die neuronalen Stammzellen wurden vor der Implantation und der Markierung mit der Probe des Beispiels 5 mit einem Gen für die Produktion eines grün-fluoreszierenden Proteins transfiziert. In der Histologie sieht man, dass die implantierten Zellen auch 16 Wochen nach Implantation noch leben und das grün fluoreszierende Protein produzieren (A, Pfeil). Dies ist in der Fluoreszenz-Mikroskopischen Untersuchung des Implantationskanals sichtbar. Eine Berliner-Blau-Eisenfärbung zeigt das Eisen (B, blaue Zellen) der erfindungsgemäßen Probe, welches vor Implantation von den Zellen aufgenommen wurde. In der Lokalisation der blauen Eisenfärbung und der grünen Fluoreszenz zeigt sich eine sehr gute Übereinstimmung.

## Patentansprüche

1. Wässrige Dispersion von superparamagnetischen Eindomänenteilchen aus Eisenhydroxid, Eisenoxidhydrat, Eisenoxid, Eisenmischoxid oder Eisen mit einer Teilchengröße von 2 bis 10 nm, die auf ihrer Oberfläche als Stabilisatorsubstanzen aliphatische Di- und/oder Tricarbonsäuren ausgewählt aus Zitronensäure, Apfelsäure, Weinsäure oder deren Gemischen tragen, **dadurch gekennzeichnet, dass** die Dispersion N-Methyl-D-glucamin (Meglumin) und/oder 2-Amino-2-(hydroxymethyl)-1,3-propandiol (Trometamol) umfasst und der freie Eisenionengehalt kleiner 1 mg/l ist.

2. Dispersion nach Anspruch 1,
**dadurch gekennzeichnet, dass**
ein physiologisch verträglicher Komplexbildner für Eisenionen enthalten ist, vorzugsweise
eine Glycerolphosphorsäure, Ethylendiamintetraessigsäure (EDTA), N-Hydroxyethyl-ethylendiamintriessigsäure (HEDTA), Diethylen-triaminpentaessigsäure (DTPA), α-Mercaptopropionylglycin (Tiopronin), 2,3-Mercapto-1-propansulfonsäure, 30-Amino-3,14,25-trihydroxy-3,9,14,20,25-pentaazatriacontan-2,10,13,21,24-penton-methansulfonsäure (Deferoxaminmesilat), ein Gemisch oder Salz davon , wobei die Kationen der Salze vorzugsweise Natrium, Kalium, Calcium ,Magnesium, D(-)-N- Methylglucamin (Meglumin), 2-Amino-2-(hydroxymethyl)-1,3-propandiol (Trometamol) oder Gemische davon sind.

3. Dispersion nach Anspruch 2, **dadurch gekennzeichnet, dass** der Komplexbildner Glycerolphosphorsäure oder ein Salz davon ist.

4. Dispersion nach einem der Ansprüche 1 bis 3,
**dadurch gekennzeichnet, dass**
sie physiologisch verträgliche polyaminogruppenhaltige Verbindungen enthält, die ausgewählt sind aus der Gruppe der Polyethylenimine (PEI), Polyvinylamine (PVAm), PEI- und PVAm- Co-Polymere, Polylysin, Spermin, Spermidin, Protamin, Protaminsulfat, Oligopeptide, Polypeptide, Denaturierungsprodukte von Proteinen und Proteiden, wie Gelatine, Kasein-Hydrolysate, Gluteline; stickstoffhaltigen Polysaccharide, wie Mucopolysaccharide, Glykoproteide, Chitine und Gemischen davon, vorzugsweise Polyethylenimine (PEI) oder Polyvinylamine (PVAm).

5. Dispersion nach Anspruch 4,
**dadurch gekennzeichnet, dass**
die polyaminogruppenhaltigen Verbindungen an diagnostisch oder pharmazeutisch wirksame Substanzen, an zell- oder gewebespezifische Substanzen, Zellen oder zellfusionsvermittelnde Substanzen oder gentransfervermittelnde Substanzen gebunden sind oder an
kurzlebige Radiopharmaka gebunden sind, die ¹¹C, ¹³N, ¹⁵O, ¹⁸F, ⁶⁸Ga, ⁷⁵Br oder ¹²³J enthalten, vorzugsweise [¹¹C] Thymidin, [¹⁸F]Fluor-L-DOPA, [⁶⁸Ga]-anti- CD66.

6. Dispersion nach einem der Ansprüche 1 bis 5, **dadurch gekennzeichnet, dass** sie positiv geladene Metallionen enthält, die aus positiv geladenen Metallionen der chemischen Elemente Kupfer, Silber, Gold, Eisen, Gallium Thallium, Bismut, Palladium, Rhenium, Ruthenium, Platin, Technetium, Indium, Iridium, Radium, Selen, Ytrium, Zirkon und seltenen Erden sowie Gemischen davon und der radioaktiven Isotope ⁵²Fe, ⁶⁷Ga, ^{99m}Tc, ¹¹³In, ¹⁸⁸Rh, ¹⁹²Ir, ¹⁹⁸Au, ²⁰¹Tl, ²²³Ra sowie Gemischen davon ausgewählt sind.

7. Verfahren zur Herstellung einer wässrigen Dispersion von superparamagnetischen Eindomänenteilchen aus Eisenhydroxid, Eisenoxidhydrat, Eisenoxid, Eisenmischoxid oder Eisen mit einer Teilchengröße von 2 bis 10 nm, die auf ihrer Oberfläche als Stabilisatorsubstanzen aliphatische Di- und/oder Tricarbonsäuren ausgewählt aus Zitronensäure, Äpfelsäure, Weinsäure oder deren Gemischen tragen, durch Fällung der superparamagnetischen Eisenteilchen aus wässrigen Eisensalzlösungen mit Alkalilauge oder Ammoniumhydroxid, anschließender Behandlung mit aliphatischen Di- und/oder Tricarbonsäuren ausgewählt aus Zitronensäure, Äpfelsäure, Weinsäure oder deren Gemischen und Reinigung der so stabilisierten Teilchen durch Dialyse mit destilliertem Wasser bis das Dialysat eine elektrische Leitfähigkeit kleiner 10 µS/cm besitzt,
**dadurch gekennzeichnet, dass**
das Dialysat nachfolgend mit einer wässrigen Salzlösung von aliphatischen Di- und/oder Tricarbonsäuren, ausgewählt aus Zitronensäure, Äpfelsäure, Weinsäure oder deren Gemischen behandelt und mit destilliertem Wasser dialysiert wird bis das Dialysat eine elektrische Leitfähigkeit kleiner 10 µS/cm und einen freien Eisenionengehalt kleiner 1 mg/l besitzt, anschließend mit einer wässrigen Lösung der genannten freien Di- und/oder Tricarbonsäuren oder deren Gemischen behandelt und mit destilliertem Wasser dialysiert wird bis das Dialysat eine elektrische Leitfähigkeit kleiner 10 µS/cm und einen freien Eisenionengehalt kleiner 1 mg/l besitzt und N-Methyl-D-glucamin (Meglumin) und/oder 2-Amino-2-(hydroxymethyl)-1,3-propandiol (Trometamol) zugesetzt wird.

8. Verfahren nach Anspruch 7,
**dadurch gekennzeichnet, dass**
der erhaltenen wässrigen Dispersion ein physiologisch verträglicher Komplex-bildner für Eisenionen zugesetzt wird, vorzugsweise eine Glycerolphosphor-säure, Ethylendiamintetraessigsäure (EDTA), N-Hydroxyethyl-ethylendiamin-triessigsäure (HEDTA), Diethylentriaminpentaessigsäure (DTPA), α-Mercapto-propionylglycin (Tiopronin), 2,3-Mercapto-1-propansulfonsäure, 30-Amino-3,14,25-trihydroxy-3,9,14,20,25-pentaazatriacontan-2,10,13,21, 24-penton-methansulfon-säure (Deferoxaminmesilat) oder ein Gemisch oder Salz davon, besonders bevorzugt eine Glycerolphosphorsäure oder ein Salz davon.

9. Verfahren nach einem der Ansprüche 7 oder 8,
**dadurch gekennzeichnet, dass**
physiologisch verträgliche polyaminogruppenhaltige Verbindungen ausgewählt aus der Gruppe der Polyethylenimine (PEI), Polyvinylamine (PVAm), PEI- und PVAm- Co-Polymere, Polylysin, Spermin, Spermidin, Protamin, Protaminsulfat, Oligopeptide, Polypeptide, Denaturierungsprodukte von Proteinen und Proteiden, wie Gelatine, Kasein-Hydrolysate, Gluteline; stickstoffhaltigen Polysaccharide, wie Mucopolysaccharide, Glykoproteide, Chitine und Gemischen davon zugesetzt werden, vorzugsweise Polyethylenimine (PEI) oder Polyvinylamine (PVAm).

10. Verfahren nach Anspruch 9,
**dadurch gekennzeichnet, dass**
an die polyaminogruppenhaltigen Verbindungen diagnostisch oder pharmazeutisch wirksame Substanzen, zell- oder gewebespezifische Bindungssubstanzen, Zellen oder zellfusionsvermittelnde Substanzen oder gentransfer-vermittelnde Substanzen gebunden werden oder
kurzlebige Radiopharmaka, die ¹¹C, ¹³N, ¹⁵O, ¹⁸F, ⁶⁸Ga, ⁷⁵Br oder ¹²³J enthalten, vorzugsweise [¹¹C] Thymidin, [¹⁸F]Fluor-L-DOPA, [⁶⁸Ga]-anti- CD66.

11. Verfahren nach einem der Ansprüche 7 bis 10,
**dadurch gekennzeichnet, dass**
der erhaltenen wässrigen Dispersion positiv geladene Metallionen zugesetzt werden, die ausgewählt sind aus positiv geladenen Metallionen der chemischen Elemente Kupfer, Silber, Gold, Eisen, Gallium Thallium, Bismut, Palladium, Rhenium, Ruthenium, Platin, Technetium, Indium, Iridium, Radium, Selen, Ytrium, Zirkon und seltenen Erden sowie Gemischen davon und der radioaktiven Isotope ⁵²Fe, ⁶⁷Ga, ^{99m}Tc, ¹¹³In, ¹⁸⁸Rh, ¹⁹²Ir, ¹⁹⁸Au, ²⁰¹Tl, ²²³Ra sowie Gemischen davon.

12. Pharmazeutische Zusammensetzung umfassend eine wässrige Dispersion von superparamagnetischen Eindomänenteilchen aus Eisenhydroxid, Eisenoxidhydrat, Eisenoxid, Eisenmischoxid oder Eisen gemäß der Ansprüche 1 bis 6 und vorzugsweise pharmazeutisch annehmbare Hilfs- und/oder Trägerstoffe .

13. Pharmazeutische Zusammensetzung nach Anspruch 12,
**dadurch gekennzeichnet, dass**
die Hilfs- und/oder Trägerstoffe Zucker, vorzugsweise Mannitol, Sorbitol, Glucose oder Xylitol, sind.

14. Verwendung der wässrigen Dispersion von superparamagnetischen Eisen-Eindomänenteilchen gemäß der Ansprüche 1 bis 6 zur Herstellung einer pharmazeutischen Zusammensetzung zur Gefäßdiagnostik, insbesondere zur Diagnostik von vulnerablen Plaques, zur Diagnostik von Primärtumoren und Metastasen und zur Diagnostik des lymphatischen Systems, insbesondere zur Auffindung des SentinelLymphknotens,
zur Herstellung einer pharmazeutischen Zusammensetzung zur parenteralen Eisenersatztherapie, Tumortherapie, Gentherapie und zum in-vivo-Zellmonitoring,
zur Herstellung einer pharmazeutischen Zusammensetzung zur Anwendung als parenterales Kontrast-mittel für die MRT in Kombination mit nuklearmedizinischen Bildgebungs-verfahren wie SZINTIGRAPHIE, SPECT oder PET oder
zur Markierung von Zellen für die Beobachtung der markierten Zellen im Körper mit der MRT in Kombination mit optischer Bildgebung oder Nuklearmedizinischer Bildgebung, vorzugsweise SZINTIGRAPHIE, SPECT oder PET.

## Claims

1. An aqueous dispersion of superparamagnetic single-domain particles of iron hydroxide, iron oxide hydrate, iron oxide, iron mixed oxide or iron with a particle size of from 2 to 10 nm, which particles bear aliphatic di- and/or tricarboxylic acids selected from citric acid, malic acid, tartaric acid or mixtures thereof as stabilizer substances on their surface, **characterized in that** the dispersion comprises N-methyl-D-glucamine (meglumine) and/or 2-amino-2-(hydroxymethyl)-1,3-propanediol (trometamol) and that the content of free iron ions is less than 1 mg/l.

2. The dispersion according to claim 1,
**characterized in that**
a physiologically tolerable complexing agent for iron ions is included, preferably glycerolphosphoric acid, ethylenediaminetetraacetic acid (EDTA), N-hydroxyethylethylenediaminetriacetic acid (HEDTA), diethylenetriaminepentaacetic acid (DTPA), α-mercaptopropionylglycine (thiopronine), 2,3-mercapto-1-propanesulfonic acid, 30-amino-3,14,25-trihydroxy-3,9,14,20,25-pentaazatriacontane-2,10,13,21,24-pentaone-methanesulfonic acid (deferoxamine mesylate), a mixture or salt thereof, the cations of the salts preferably being sodium, potassium, calcium, magnesium, D-(-)-N-methylglucamine (meglumine), 2-amino-2-(hydroxymethyl)-1,3-propandiol (trometamol) or mixtures thereof.

3. The dispersion according to claim 2,
**characterized in that**
the complexing agent is glycerolphosphoric acid or a salt thereof.

4. The dispersion according to any of claims 1 to 3,
**characterized in that**
it includes physiologically tolerable compounds containing polyamino groups, selected from the group of polyethyleneimines (PEI), polyvinylamines (PVAm), PEI and PVAm copolymers, polylysine, spermine, spermidine, protamin, protamin sulfate, oligopeptides, polypeptides, denaturation products of proteins and proteids, such as gelatin, casein hydrolyzates, glutelins; nitrogen-containing polysaccharides such as mucopolysaccharides, glycoproteids, chitins and mixtures thereof, preferably polyethyleneimines (PEI) or polyvinylamines (PVAm).

5. The dispersion according to claim 4,
**characterized in that**
the compounds containing polyamino groups are bound to diagnostically or pharmaceutically effective substances, cell- or tissue-specific substances, cells or cell fusion-mediating substances or gene transfer-mediating substances or to short-lived radiopharmaceutical agents containing ¹¹C, ¹³N, ¹⁵O, ¹⁸F, ⁶⁸Ga, ⁷⁵Br, ¹²³I, preferably [¹¹C]-thymidine, [¹⁸F]-fluoro-L-DOPA, [⁶⁸Ga]-anti-CD66.

6. The dispersion according to any of claims 1 to 5,
**characterized in that**
it contains positively charged metal ions selected from positively charged metal ions of the chemical elements copper, silver, gold, iron, gallium, thallium, bismuth, palladium, rhenium, ruthenium, platinum, technetium, indium, iridium, radium, selenium, yttrium, zirconium and rare earths, as well as mixtures thereof, and of the radioactive isotopes ⁵²Fe, ⁶⁷Ga, ^{99m}Tc, ¹¹³In, ¹⁸⁸Rh, ¹⁹²Ir, ¹⁹⁸Au, ²⁰¹Tl , ²²³Ra, as well as mixtures thereof.

7. A method for the production of an aqueous dispersion of superparamagnetic single-domain particles of iron hydroxide, iron oxide hydrate, iron oxide, iron mixed oxide or iron with a particle size of from 2 to 10 nm, which particles bear aliphatic di- and/or tricarboxylic acids selected from citric acid, malic acid, tartaric acid or mixtures thereof as stabilizer substances on their surface, by precipitation of the superparamagnetic iron-containing particles from aqueous iron salt solutions using an alkali solution or ammonium hydroxide, subsequent treatment with aliphatic di- and/or tricarboxylic acids selected from citric acid, malic acid and tartaric acid or mixtures thereof, and purification of the particles thus stabilized using dialysis with distilled water until the dialyzate has an electric conductivity of less than 10 µS/cm,
**characterized in that**
the dialyzate is subsequently treated with an aqueous salt solution of aliphatic di- and/or tricarboxylic acids selected from citric acid, malic acid, tartaric acid or mixtures thereof and dialyzed with distilled water until the dialyzate has an electric conductivity of less than 10 µS/cm and a content of free iron ions of less than 1 mg/l, subsequently treated with an aqueous solution of the above-mentioned free di- and/or tricarboxylic acids or mixtures thereof and dialyzed with distilled water until the dialyzate has an electric conductivity of less than 10 µS/cm and a content of free iron ions of less than 1 mg/l, and N-methyl-D-glucamine (meglumine) and/or 2-amino-2-(hydroxymethyl)-1,3-propanediol (trometamol) are added.

8. The method according to claim7,
**characterized in that**
the resulting aqueous dispersion is added with a physiologically tolerable iron ions complexing agent, preferably glycerolphosphoric acid, ethylenediaminetetraacetic acid (EDTA), N-hydroxyethylethylenediaminetriacetic acid (HEDTA), diethylenetriaminepentaacetic acid (DTPA), α-mercaptopropionylglycine (thiopronine), 2,3-mercapto-1-propanesulfonic acid, 30-amino-3,14,25-trihydroxy-3,9,14,20,25-pentaazatriacontane-2,10,13,21,24-pentaone-methanesulfonic acid (deferoxamine mesylate) or a mixture or salt thereof, more preferably glycerolphosphoric acid or a salt thereof.

9. The method according to any of claims 7 or 8,
**characterized in that**
physiologically tolerable compounds containing polyamino groups, selected from the group of polyethyleneimines (PEI), polyvinylamines (PVAm), PEI and PVAm copolymers, polylysine, spermine, spermidine, protamin, protamin sulfate, oligopeptides, polypeptides, denaturation products of proteins and proteids, such as gelatin, casein hydrolyzates, glutelins; nitrogen-containing polysaccharides such as mucopolysaccharides, glycoproteids, chitins and mixtures thereof, are added, preferably polyethyleneimines (PEI) or polyvinylamines (PVAm).

10. The method according to claim 9,
**characterized in that**
diagnostically or pharmaceutically effective substances, cell- or tissue-specific binding substances, cells or cell fusion-mediating substances or gene transfer-mediating substances or short-lived radiopharmaceutical agents containing ¹¹C, ¹³N, ¹⁵O, ¹⁸F, ⁶⁸Ga, ⁷⁵Br, ¹²³I, preferably [¹¹C]-thymidine, [¹⁸F]-fluoro-L-DOPA, [⁶⁸Ga]-anti-CD66 are bound to the compounds containing polyamino groups.

11. The method according to any of claims 7 to 10,
**characterized in that**
the resulting aqueous dispersion is added with positively charged metal ions selected from positively charged metal ions of the chemical elements copper, silver, gold, iron, gallium, thallium, bismuth, palladium, rhenium, ruthenium, platinum, technetium, indium, iridium, radium, selenium, yttrium, zirconium and rare earths, as well as mixtures thereof, and of the radioactive isotopes ⁵²Fe, ⁶⁷Ga, ^{99m}Tc, ¹¹³In, ¹⁸⁸Rh, ¹⁹²Ir, ¹⁹⁶Au, ²⁰¹Tl, ²²³Ra, as well as mixtures thereof.

12. A pharmaceutical composition comprising an aqueous dispersion of superparamagnetic single-domain particles of iron hydroxide, iron oxide hydrate, iron oxide, iron mixed oxide or iron in accordance with claims 1 to 6 and preferably pharmaceutically acceptable adjuvants and/or vehicles.

13. The pharmaceutical composition according to claim 12,
**characterized in that**
the adjuvants and/or vehicles are sugars, preferably mannitol, sorbitol, glucose or xylitol.

14. Use of the aqueous dispersion of superparamagnetic iron single-domain particles according to claims 1 to 6 in the production of a pharmaceutical composition for vascular diagnostics, especially for the diagnostics of vulnerable plaques, for the diagnostics of primary tumors and metastases, and for the diagnostics of the lymphatic system, particularly for the detection of the sentinel lymph node,
in the production of a pharmaceutical composition for parenteral iron replacement therapy, tumor therapy, gene therapy and *in vivo* cell monitoring. in the production of a pharmaceutical composition for use as parenteral contrast medium for MRT in combination with nuclear-medical imaging methods such as scintigraphy, SPECT or PET,
in cell labeling to observe the labeled cells in the body using MRT in combination with optical imaging or nuclear-medical imaging, preferably scintigraphy, SPECT or PET.

## Revendications

1. Dispersion aqueuse de particules superparamagnétiques mono-domaines d'hydroxyde de fer, d'oxyde de fer hydraté, d'oxyde de fer, d'oxyde mixte de fer ou de fer ayant une granulométrie de 2 à 10 nm, qui portent sur leur surface, à titre de substances stabilisantes, des acides di- et/ou tricarboxyliques aliphatiques sélectionnés parmi l'acide citrique, l'acide malique, l'acide tartrique ou des mélanges de ceux-ci, **caractérisée en ce que** la dispersion comprend du N-méthyl-D-glucamine (méglumine) et/ou du 2-amino-2-(hydroxyméthyl)-1,3-propanediol (trométamol) et que la teneur en ions libres de fer est inférieure à 1 mg/l.

2. Dispersion selon la revendication 1,
**caractérisée en ce**
**qu'**elle contient un agent complexant pour les ions de fer physiologiquement tolérable,
de préférence de l'acide glycérolphosphorique, de l'acide éthylènediaminetétraacétique (EDTA), de l'acide N-hydroxyéthyléthylènediaminetriacétique (HEDTA), de l'acide diéthylène-triaminepentaacétique (DTPA), de la α-mercaptopropionylglycine (thiopronine), de l'acide 2,3-mercapto-1-propanesulfonique, de l'acide 30-amino-3,14,25-trihydroxy-3,9,14,20,25-pentaazatriacontane-2,10,13,21,24-pentaone-méthanesulfonique (déféroxamine mesylate), un mélange ou un sel de ceux-ci, les cations des sels étant de préférence du sodium, du potassium, du calcium, du magnésium, du D(-)-N-méthylglucamine (méglumine), du 2-amino-2-(hydroxyméthyl)-1,3-propanediol (trométamol) ou des mélanges de ceux-ci.

3. Dispersion selon la revendication 2,
**caractérisée en ce que**
l'agent complexant est de l'acide glycérolphosphorique ou un sel de celui-ci.

4. Dispersion selon l'une quelconque des revendications 1 à 3,
**caractérisée en ce**
**qu'**elle comprend des composés physiologiquement tolérables contenant des groupes polyamino, qui sont choisis parmi le groupe comprenant les polyéthylèneimines (PEI), polyvinylamines (PVAm), les copolymères de PEI et PVAm, la polylysine, la spermine, la spermidine, le protamine, le protamine sulfate, les oligopeptides, les polypeptides, les produits de dénaturation des protéines et des protéides, tels que la gélatine, les hydrolysats de caséine, les glutélines, les polysaccharides azotés tels que les mucopolysaccharides, les glycoprotéides, les chitines et les mélanges de ceux-ci, de préférence des polyéthylèneimines (PEI) ou des polyvinylamines (PVAm).

5. Dispersion selon la revendication 4,
**caractérisée en ce que**
les composés contenant des groupes polyamino sont liés à des substances diagnostiquement ou pharmaceutiquement efficaces, des substances spécifiques à des cellules ou à des tissus, des substances médiatrices de la fusion cellulaire ou des cellules ou des substances médiatrices de transfert de gène,
ou sont liés à des
agents radiopharmaceutiques à courte vie contenant du ¹¹C, ¹³N, ¹⁵O, ¹⁸F, ⁶⁸Ga, ⁷⁵Br ou ¹²³I, de préférence de la [¹¹C]-thymidine, du [¹⁸F]-fluoro-L-DOPA, du [⁶⁸Ga]-anti-CD66.

6. Dispersion selon l'une quelconque des revendications 1 à 5, **caractérisée en ce qu'**elle contient des ions métalliques à charge positive sélectionnés parmi les ions métalliques chargés positivement des éléments chimiques cuivre, argent, or, fer, gallium, thallium, bismuth, palladium, rhénium, ruthénium, platine, technétium, indium, iridium, radium, sélénium, yttrium, zirconium et terres rares, et des mélanges de ceux-ci, et les isotopes radioactifs ⁵²Fe, ⁶⁷Ga, ^{99m}Tc, ¹¹³In, ¹⁸⁸Rh, ¹⁹²Ir, ¹⁹⁶Au, ²⁰¹Tl, ²²³Ra, et des mélanges de ceux-ci.

7. Procédé de fabrication d'une dispersion aqueuse de particules superparamagnétiques mono-domaines d'hydroxyde de fer, d'oxyde de fer hydraté, d'oxyde de fer, d'oxyde mixte de fer ou de fer ayant une granulométrie de 2 à 10 nm, qui portent sur leur surface, à titre de substances stabilisantes, des acides di- et/ou tricarboxyliques aliphatiques sélectionnés parmi l'acide citrique, l'acide malique, l'acide tartrique ou des mélanges de ceux-ci, par précipitation des particules superparamagnétiques contenant du fer à partir de solutions aqueuses de sel de fer en utilisant une solution alcaline ou de l'hydroxyde d'ammonium, traitement suivant avec des acides di- et/ou tricarboxyliques aliphatiques sélectionnés parmi l'acide citrique, l'acide malique, l'acide tartrique ou des mélanges de ceux-ci, et purification des particules ainsi stabilisées par dialyse avec de l'eau distillée jusqu'à ce que le dialysat obtenu présente une conductivité électrique inférieure à 10 µS/cm, **caractérisé en ce que**
le dyalisat est traité ensuite avec une solution aqueuse de sel d'acides di- et/ou tricarboxyliques aliphatiques sélectionnés parmi l'acide citrique, l'acide malique, l'acide tartrique ou des mélanges de ceux-ci et dialysé avec de l'eau distillée jusqu'à ce que le dialysat ait une conductivité électrique inférieure à 10 µS/cm et une teneur en ions libres de fer inférieure à 1 mg/l, puis traité avec une solution aqueuse des acides libres di- et/ou tricarboxyliques susmentionnés ou des mélanges de ceux-ci, et dialysé à nouveau avec de l'eau distillée jusqu'à ce que le dialysat ait une conductivité électrique inférieure à 10 µS/cm et une teneur en ions libres de fer inférieure à 1 mg/l, et on ajoute enfin du N-méthyl-D-glucamine (méglumine) et/ou du 2-amino-2-(hydroxyméthyl)-1,3-propanediol (trométamol).

8. Procédé selon la revendication 7,
**caractérisé en ce**
**qu'**on ajoute à la dispersion aqueuse obtenue un agent complexant pour les ions de fer physiologiquement tolérable, de préférence de l'acide glycérolphosphorique, de l'acide éthylènediaminetétraacétique (EDTA), de l'acide N-hydroxyéthyl-éthylènediaminetriacétique (HEDTA), de l'acide diéthylène-triaminepentaacétique (DTPA), de la α-mercaptopropionylglycine (thiopronine), de l'acide 2,3-mercapto-1-propanesulfonique, de l'acide 30-amino-3,14,25-trihydroxy-3,9,14,20,25-pentaazatriacontane-2,10,13,21,24-penta-one-méthanesulfonique (déféroxamine mésylate) ou un mélange ou un sel de ceux-ci, et de manière plus préférée de l'acide glycérolphosphorique ou un sel de celui-ci.

9. Procédé selon l'une des revendications 7 ou 8,
**caractérisé en ce**
**qu'**on ajoute des composés physiologiquement tolérables contenant des groupes polyamino, choisis parmi le groupe comprenant les polyéthylèneimines (PEI), polyvinylamines (PVAm), les copolymères de PEI et PVAm, la polylysine, la spermine, la spermidine, le protamine, le protamine sulfate, les oligopeptides, les polypeptides, les produits de dénaturation des protéines et des protéides, tels que la gélatine, les hydrolysats de caséine, les glutélines, des polysaccharides azotés tels que les mucopolysaccharides, les glycoprotéides, les chitines et les mélanges de ceux-ci, de préférence des polyéthylèneimines (PEI) ou des polyvinylamines (PVAm).

10. Procédé selon la revendication 9,
**caractérisé en ce que**
des substances diagnostiquement ou pharmaceutiquement efficaces, des substances spécifiques à des cellules ou à des tissus, ou des substances médiatrices de la fusion cellulaire ou des cellules ou des substances médiatrices du transfert de gènes, ou
des agents radiopharmaceutiques à courte vie contenant du ¹¹C, ¹³N, ¹⁵O, ¹⁸F, ⁶⁸Ga, ⁷⁵Br ou ¹²³I, de préférence de la [¹¹C]-thymidine, du [¹⁸F]-fluoro-L-DOPA, du [⁶⁸Ga]-anti-CD66,
sont liés aux composés contenant des groupes polyamino.

11. Procédé selon l'une quelconque des revendications 7 à 10,
**caractérisé en ce que**
des ions métalliques à charge positive sélectionnés parmi les ions métalliques chargés positivement des éléments chimiques cuivre, argent, or, fer, gallium, thallium, bismuth, palladium, rhénium, ruthénium, platine, technétium, indium, iridium, radium, sélénium, yttrium, zirconium et terres rares, et des mélanges de ceux-ci, et les isotopes radioactifs ⁵²Fe, ⁶⁷Ga, ^{99m}Tc, ¹¹³In, ¹⁸⁸Rh, ¹⁹²Ir, ¹⁹⁸Au, ²⁰¹Tl, ²²³Ra, et les mélanges de ceux-ci, sont ajoutés à la dispersion aqueuse obtenue.

12. Composition pharmaceutique comprenant une dispersion aqueuse de particules superparamagnétiques mono-domaines d'hydroxyde de fer, d'oxyde de fer hydraté, d'oxyde de fer, d'oxyde mixte de fer ou de fer selon les revendications 1 à 6 et de préférence des adjuvants et/ou des vecteurs pharmaceutiquement acceptables.

13. Composition pharmaceutique selon la revendication 12,
**caractérisée en ce que**
les adjuvants et/ou les vecteurs sont des sucres, de préférence du mannitol, du glucose ou du xylitol.

14. Utilisation de la dispersion aqueuse de particules de fer superparamagnétiques mono-domaines selon les revendications 1 à 6 pour préparer une composition pharmaceutique destinée au diagnostic des vaisseaux, notamment au diagnostic des plaques vulnérables, au diagnostic des tumeurs primaires et des métastases, et au diagnostic du système lymphatique, en particulier à la détection du noeud lymphatique sentinelle,
pour préparer une composition pharmaceutique destinée à une thérapie de remplacement du fer par voie parentérale, une thérapie tumorale, une thérapie génique et une surveillance *in vivo* des cellules,
pour préparer une composition pharmaceutique destinée à être utilisée en tant qu'agent de contraste parentéral pour la TRM en association avec des méthodes d'imagerie de médecine nucléaire telles que la scintigraphie, la SPECT ou la PET, ou
pour le marquage des cellules dans le but d'observer les cellules marquées dans le corps en utilisant la TRM en association avec une imagerie optique ou une imagerie de médecine nucléaire, de préférence la scintigraphie, la SPECT ou PET.
